# EUROPEAN PATENT APPLICATION

(11) **EP 4 567 029 A1**
(43) Date of publication of application: **11.06.2025**
(21) Application number: 23849469.4
(22) Date of filing: 02.08.2023
(51) Int. Cl.: C07D 239/54, A01N 43/54, A01P 13/00

(54) **METHOD FOR PREPARING URACIL COMPOUND CONTAINING CARBOXYLATE FRAGMENT**

(30) Priority: 04.08.2022 CN 202210934903
(71) Applicant: Jiangsu Flag Chemical Industry Co., Ltd., Nanjing, Jiangsu 210000 (CN)
(72) Inventor: ZHANG, Pu, Nanjing, Jiangsu 210000 (CN); YAO, Kaicheng, Nanjing, Jiangsu 210000 (CN); LUO, Wen, Nanjing, Jiangsu 210000 (CN); BU, Long, Nanjing, Jiangsu 210000 (CN); QIAN, Ping, Nanjing, Jiangsu 210000 (CN); WU, Yaojun, Nanjing, Jiangsu 210000 (CN)
(74) Representative: Sun, Yanan
(86) International application number: PCT/CN2023/110770
(87) International publication number: WO 2024/027765

(57) **Abstract**

The present invention relates to the field of herbicides, in particular to a preparation method for a 2-chloro-4-fluoro-5-(3-methyl-2,6-dioxo-4-(trifluoromethyl)-3,6-dihydropyrimidin-1(2H)-yl) benzoate compound of formula (I). The present invention constructs a uracil ring under an acidic condition, and the intermediate prepared by this method can be used to prepare a novel high-efficiency uracil herbicide of structural formula (I), which has stable chemical properties and better herbicidal activity. The synthesis route is simple, and has broad application prospects in agriculture. The present invention further provides intermediate compounds used in the method, as well as methods for producing the intermediate compounds.

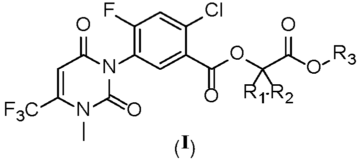

## Description

### TECHNICAL FIELD

The present invention relates to the field of herbicides, in particular to a method for preparing a uracil compound containing a carboxylate fragment. Such uracil compound can be used to prepare 2-chloro-4-fluoro-5-(3-methyl-2,6-dioxo-4-(trifluoromethyl)-3,6-dihydropyrimidin-1(2H)-yl) benzoate compound.

### BACKGROUND

A preparation method for a uracil compound containing carboxylate fragments is already known from CN114621150A:

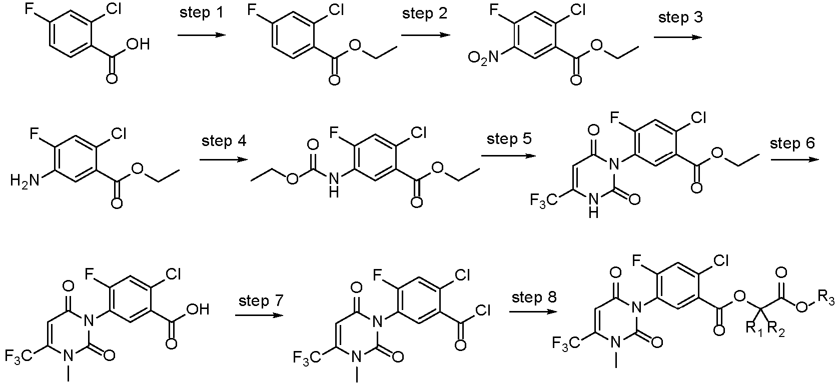

The following preparation method is also known from CN114621150A:

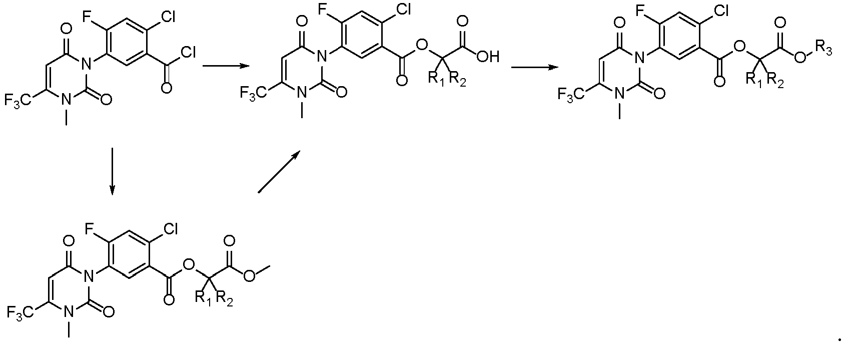

In the above scheme:
R₁ and R₂ are each independently selected from hydrogen or methyl; or R₁ and R₂ together with the carbon atom to which they are attached form a three-membered carbocycle;
R₃ is selected from C₂₋₅ alkyl, CH₃CH=CHCHCH₂-, C₃₋₆ alkenyl substituted by one or more halogens, CH₃C≡CCH₂-, C₃₋₆ alkynyl substituted by one or more halogens, C₄₋₇ cycloalkyl, C₃₋₆ cycloalkyl-C₁₋₃ alkyl, or C₁₋₆ halogen-substituted alkyl;
when R₁ is selected from hydrogen and R₂ is selected from methyl, the chiral carbon atom connected thereto is selected from either an R configuration or an S configuration, or a mixture of the two.

The existing methods for preparing uracil compound herbicides mostly use esterification protection of carboxyl before synthesizing a uracil ring, followed by one-step hydrolysis, and then splicing with corresponding fragment to synthesize the final compound. The hydrolysis step conditions of this method require high temperature reaction under strong acid conditions. The reaction conditions are intense, which is easy to produce impurities. At the same time, the material requirements of the reaction device are relatively strict. Most hydrolysis methods use hydrochloric acid-acetic acid system, which produces a large amount of waste acid that is difficult to recycle. The strong acid is corrosive, which is prone to danger during operation. There are problems such as poor atom economy benefits, long steps, high cost, low yield, non-environmental protection, and difficult post-treatment. At present, the key step in the preparation of a uracil compound herbicide is the synthesis of the uracil ring. Most of the reported methods for synthesizing the ring of the uracil compound herbicide are relatively single, which is not conducive to industrial development.

### SUMMARY

The technical problem to be solved by the present invention is to provide a method for preparing a uracil compounds containing a carboxylate fragment, in response to the drawbacks of the prior art.

The technical solution of the present invention to solve the above technical problem is to provide a method for preparing a uracil compound containing a carboxylate fragment with the following reaction scheme:

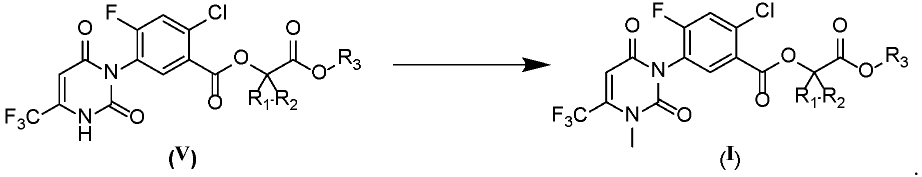

The compound of formula (V) reacts with a methylation reagent in the presence of a base in an organic solvent at -20 °C to the boiling point of the solvent to give a uracil compound of formula (I);
wherein,
R₁ and R₂ are each independently selected from hydrogen or methyl; or R₁ and R₂ together with the carbon atom to which they are attached form a three-membered carbocycle;
R₃ is selected from C_{1∼3} alkoxy-C_{1~3} alkyl, C_{1∼3} halogenated alkoxy-C_{1~3} alkyl, C_{2~6} enyloxy-C_{1∼3} alkyl, C_{2~6} halogenated enyloxy-C_{1~3} alkyl, C_{2~6} ynyloxy-C_{1∼3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

Preferably, in the above synthesis method, R₁ and R₂ are each independently selected from hydrogen or methyl; or R₁ and R₂ together with the carbon atom to which they are attached form a three-membered carbocycle, and the three-membered carbocycle is cyclopropyl;
R₃ is selected from C_{1∼3} alkoxy-C_{1~3} alkyl, C_{1∼3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.
the organic solvent is selected from pentane, n-hexane, cyclohexane, heptane, octane, dichloromethane, 1,2-dichloroethane, chloroform, carbon tetrachloride, petroleum ether, ether, methyl tert-butyl ether, ethylene glycol dimethyl ether, tetrahydrofuran, 1,4-dioxane, ethyl acetate, butyl acetate, benzene, toluene, o-xylene, m-xylene, p-xylene, xylene, chlorobenzene, acetone, butanone, 4-methyl-2-pentanone, cyclohexanone, N-methylpyrrolidone, acetonitrile, N, N-dimethylformamide, N, N-dimethylacetamide, or dimethyl sulfoxide;
the base is selected from 4-dimethylaminopyridine, triethylamine, diisopropylethylamine, pyridine, 2-methylpyridine, 1,8-diazabicyclo[5.4.0]undec-7-ene(DBU), 2,6-Lutidine, NaH, NaNH₂, NaHCO₃, Na₂CO₃, K₂CO₃, KHCO₃, Cs₂CO₃, NaOH, LiOH, or KOH;
the amount of the base is 1.0 to 3.0 equivalents;
the methylation reagent is selected from dimethyl sulfate, chloromethane, methyl bromide, methyl iodide, methyl p-toluenesulfonate and methyl trifluoromethanesulfonate; and
the reaction temperature is 0 °C to room temperature.

More preferably, in the above synthesis method, the organic solvent is N,N-dimethylformamide, the base is K₂CO₃, the amount of the base is 2.0 and 2.5 equivalents, the methylation reagent is selected from dimethyl sulfate, methyl bromide, methyl iodide, and the reaction temperature is room temperature.

Further, the present invention also provides a method for preparing a uracil compound containing a carboxylate fragment of formula (V) with the following reaction scheme:

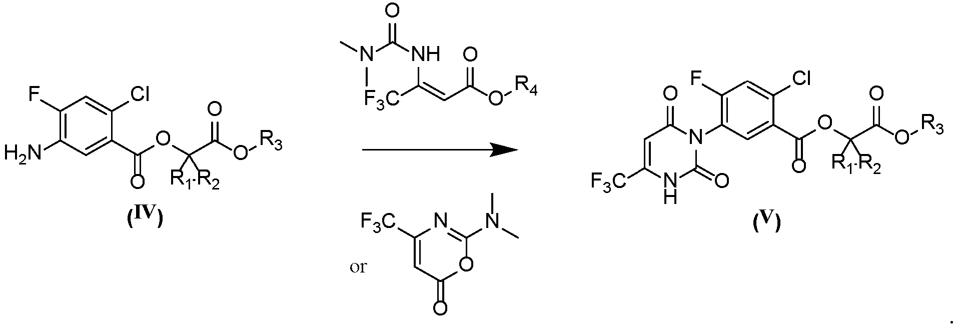

The compound of formula (IV) reacts with R₄ 3-(3,3-dimethylureido)-4,4,4-trifluorobut-2-enoate or 2-(dimethylamino)-4-(trifluoromethyl)-6H-1,3-oxazin-6-one in an organic solvent at -20 °C to the boiling point of the solvent in the presence of an acid to give a compound of formula (V);
wherein,
R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two; and
R₄ is C_{1∼4} alkyl.

Preferably, in the above synthesis method, R₁ and R₂ are each independently selected from hydrogen or methyl; or R₁ and R₂ together with the carbon atom to which they are attached form a three-membered carbocycle, and the three-membered carbocycle is cyclopropyl;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.
R₄ is selected from methyl or ethyl;
the organic solvent is selected from acetic acid, methanol, ethanol, isopropanol, butanol, tert-butanol, cyclohexanol, ethylene glycol, pentane, n-hexane, cyclohexane, heptane, octane, dichloromethane, 1,2-dichloroethane, chloroform, carbon tetrachloride, petroleum ether, ether, methyl tert-butyl ether, ethylene glycol dimethyl ether, tetrahydrofuran, 1,4-dioxane, ethyl acetate, butyl acetate, benzene, toluene, o-xylene, m-xylene, p-xylene, xylene, chlorobenzene, acetone, butanone, 4-methyl-2-pentanone, cyclohexanone, N-methylpyrrolidone, acetonitrile, N,N-dimethylformamide, N,N-dimethylacetamide, or dimethyl sulfoxide;
the acid is selected from formic acid, acetic acid, trifluoroacetic acid, p-toluenesulfonic acid, sulfuric acid, hydrochloric acid, phosphoric acid, or nitric acid;
the amount of the acid is 3.0 to 10.0 equivalents; and
the reaction temperature is room temperature to the boiling point of the solvent.

More preferably, in the above synthesis method, the organic solvent is acetic acid, the acid is acetic acid, the amount of the acid is 4.0 to 6.0 equivalents, and the reaction temperature is 110°C.

Furthermore, the present invention also provides a method for preparing an aniline compound containing a carboxylate fragment of formula (IV) with the following reaction scheme:

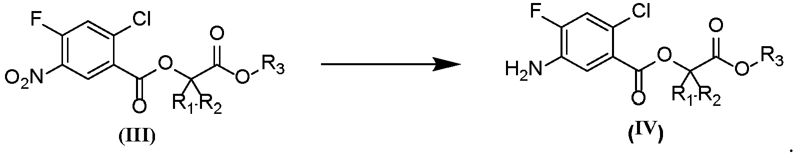

The compound of formula (III) reacts with a reductant in water or an organic solvent at -20 °C to the boiling point of the solvent to give a compound of formula (IV);
wherein,
R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2; when R₁ is selected from hydrogen and R₂ is selected from methyl, the chiral carbon atom connected thereto is selected from either an R configuration or an S configuration, or a mixture of the two.

Preferably, in the above synthesis method, R₁ and R₂ are each independently selected from hydrogen or methyl; or R₁ and R₂ together with the carbon atom to which they are attached form a three-membered carbocycle, and the three-membered carbocycle is cyclopropyl;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1∼3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1;
the solvent is selected from water, methanol, ethanol, isopropanol, butanol, tert-butanol, cyclohexanol, ethylene glycol, pentane, n-hexane, cyclohexane, heptane, octane, dichloromethane, 1,2-dichloroethane, chloroform, carbon tetrachloride, petroleum ether, ether, methyl tert-butyl ether, ethylene glycol dimethyl ether, tetrahydrofuran, 1,4-dioxane, ethyl acetate, butyl acetate, benzene, toluene, o-xylene, m-xylene, p-xylene, xylene, chlorobenzene, acetone, butanone, 4-methyl-2-pentanone, cyclohexanone, N-methylpyrrolidone, acetonitrile, N,N-dimethylformamide, N,N-dimethylacetamide, or dimethyl sulfoxide;
the reductant is selected from hydrogen, metal hydride, semimetal hydride and its derivatives such as lithium aluminum hydride, diisobutyl aluminum hydride, sodium borohydride, borane, etc., preferably hydrogen with a pressure of 1.5-2MPa; at the same time, hydrogen can be provided by hydrogen storage cylinders, or be generated in situ by active metals (such as reduced iron powder, reduced zinc powder, etc.) under acidic conditions (such as hydrochloric acid, sulfuric acid) which participate in the reduction reaction; in the catalytic amount of transition metals or catalytic amount of transition metal compounds, transition metals can be the eighth subgroup elements, preferably Ni, Pd, Pt, etc.(used directly or loaded by activated carbon, aluminum oxide, silicon dioxide or other media), more preferably Pt/C (1%), with a feeding ratio 1 wt%-5 wt% of the compound of formula (III);
the reaction temperature is room temperature to the boiling point of the solvent; and
the reaction time is 0.5-48h.

Preferably, in the above synthesis method, R₁ and R₂ are each independently selected from hydrogen or methyl;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1∼3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1;
the solvent is methanol;
the reductant is hydrogen;
the catalyst is Pt/C, with a feeding ratio 1 wt%-5 wt% of the compound of formula (III);
the reaction temperature is 40~45 °C; and
the reaction time is 8∼10h.

Furthermore, the present invention also provides a method for preparing a nitrobenzene compound containing a carboxylate fragment of formula (III) with the following reaction scheme:

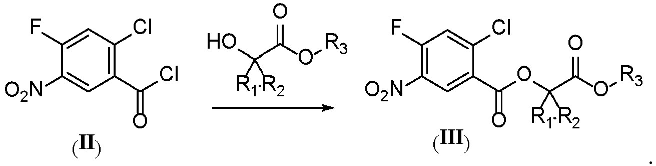

The compound of formula (II) reacts with a substituted hydroxyacetate in the presence of a base in an organic solvent at -20 °C to the boiling point of the solvent to give a compound of formula (III);
wherein,
R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

Preferably, in the above synthesis method, R₁ and R₂ are each independently selected from hydrogen or methyl;
R₃ is selected from C_{1∼3} alkoxy-C_{1~3} alkyl, C_{1∼3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1;
the organic solvent is selected from pentane, n-hexane, cyclohexane, heptane, octane, dichloromethane, 1,2-dichloroethane, chloroform, carbon tetrachloride, petroleum ether, ether, methyl tert-butyl ether, ethylene glycol dimethyl ether, tetrahydrofuran, 1,4-dioxane, ethyl acetate, butyl acetate, benzene, toluene, o-xylene, m-xylene, p-xylene, xylene, chlorobenzene, acetone, butanone, 4-methyl-2-pentanone, cyclohexanone, N-methylpyrrolidone, acetonitrile, N,N-dimethylformamide, N,N-dimethylacetamide, or dimethyl sulfoxide;
the base is selected from 4-dimethylaminopyridine, triethylamine, diisopropylethylamine, pyridine, 2-methylpyridine, 1,8-diazabicyclo[5.4.0]undec-7-ene(DBU), 2,6-Lutidine, NaH, NaNH₂, NaHCO₃, Na₂CO₃, K₂CO₃, KHCO₃, Cs₂CO₃, NaOH, LiOH, or KOH;
the amount of the base is 1.0 to 2.0 equivalents; and
the reaction temperature is 0 °C to solvent reflux temperature.

More preferably, in the above synthesis method, the organic solvent is selected from dichloromethane and toluene;
the base is selected from pyridine, triethylamine, 4-dimethylaminopyridine;
the amount of the base is 1.0 to 1.5 equivalents; and
the reaction temperature is 0~25°C or 80~90°C.

The present invention also provides a uracil compound containing a carboxylate fragment of formula (V): 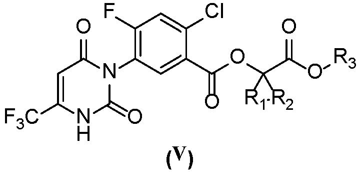
wherein R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

Preferably, in the uracil compound containing a carboxylate fragment of formula (V), R₁ and R₂ are each independently selected from hydrogen or methyl; or R₁ and R₂ together with the carbon atom to which they are attached form a three-membered carbocycle, and the three-membered carbocycle is cyclopropyl;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1∼3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

Some intermediate compounds of the present invention can be described using the specific compounds listed in Table 1, but the present invention is not limited to these compounds.

**Table 1 Structure of some compounds with general formula (V)**

| NO. | R₁ | R₂ | R₃ |
|---|---|---|---|
| **V-1** | H | H | 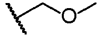 |
| **V-2** | H | H | 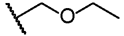 |
| **V-3** | H | H | 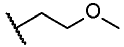 |
| **V-4** | H | H | 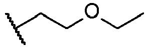 |
| **V-5** | H | H | 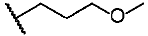 |
| **V-6** | H | H | 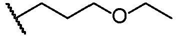 |
| **V-7** | H | H | 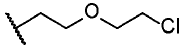 |
| **V-8** | H | H | 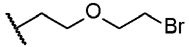 |
| **V-9** | H | H | 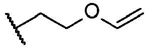 |
| **V-10** | H | H | 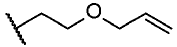 |
| **V-11** | H | H | 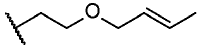 |
| **V-12** | H | H | 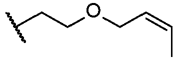 |
| **V-13** | H | H | 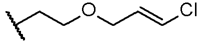 |
| **V-14** | H | H | 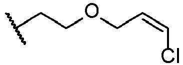 |
| **V-15** | H | H | 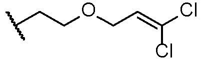 |
| **V-16** | H | H | 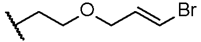 |
| **V-17** | H | H | 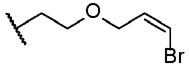 |
| **V-18** | H | H | 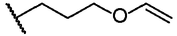 |
| **V-19** | H | H | 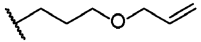 |
| **V-20** | H | H | 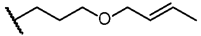 |
| **V-21** | H | H | 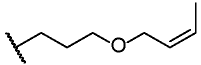 |
| **V-22** | H | H | 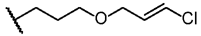 |
| **V-23** | H | H | 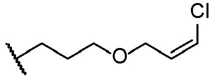 |
| **V-24** | H | H | 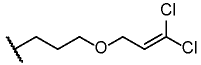 |
| **V-25** | H | H | 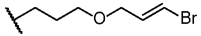 |
| **V-26** | H | H | 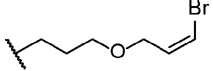 |
| **V-27** | H | H | 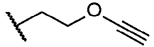 |
| **V-28** | H | H | 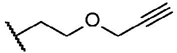 |
| **V-29** | H | H | 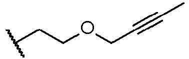 |
| **V-30** | H | H | 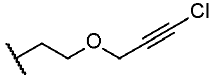 |
| **V-31** | H | H | 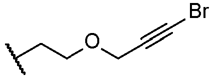 |
| **V-32** | H | H | 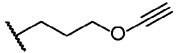 |
| **V-33** | H | H | 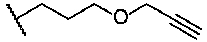 |
| **V-34** | H | H | 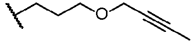 |
| **V-35** | H | H | 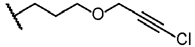 |
| **V-36** | H | H | 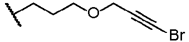 |
| **V-37** | H | H | 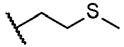 |
| **V-38** | H | H | 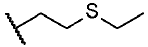 |
| **V-39** | H | H | 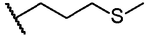 |
| **V-40** | H | H | 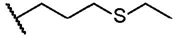 |
| **V-41** | H | H | 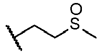 |
| **V-42** | H | H | 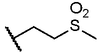 |
| **V-43** | H | H | 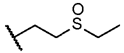 |
| **V-44** | H | H | 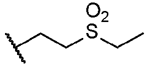 |
| **V-45** | H | 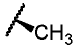 | 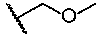 |
| **V-46** | H | 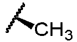 | 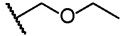 |
| **V-47** | H | 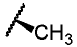 | 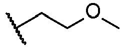 |
| **V-48** | H | 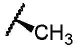 | 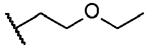 |
| **V-49** | H | 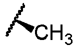 | 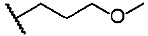 |
| **V-50** | H | 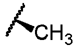 | 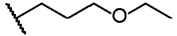 |
| **V-51** | H | 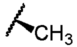 | 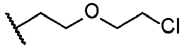 |
| **V-52** | H | 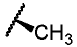 | 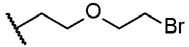 |
| **V-53** | H | 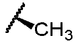 | 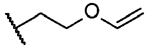 |
| **V-54** | H | 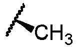 | 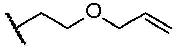 |
| **V-55** | H | 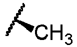 | 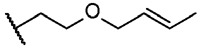 |
| **V-56** | H | 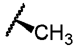 | 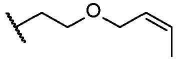 |
| **V-57** | H | 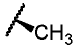 | 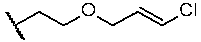 |
| **V-58** | H | 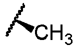 | 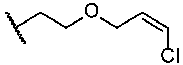 |
| **V-59** | H | 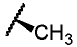 | 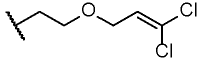 |
| **V-60** | H | 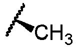 | 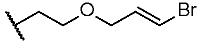 |
| **V-61** | H | 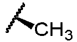 | 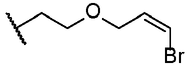 |
| **V-62** | H | 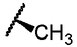 | 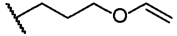 |
| **V-63** | H | 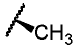 | 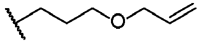 |
| **V-64** | H | 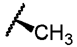 | 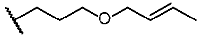 |
| **V-65** | H | 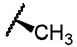 | 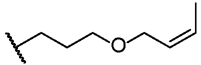 |
| **V-66** | H | 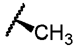 | 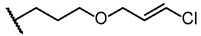 |
| **V-67** | H | 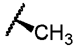 | 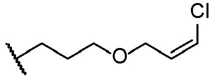 |
| **V-68** | H | 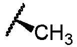 | 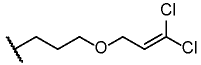 |
| **V-69** | H | 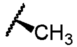 | 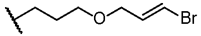 |
| **V-70** | H | 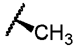 | 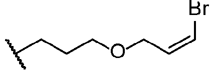 |
| **V-71** | H | 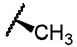 | 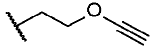 |
| **V-72** | H | 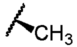 | 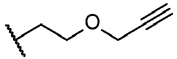 |
| **V-73** | H | 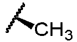 | 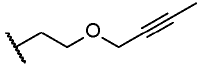 |
| **V-74** | H | 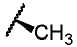 | 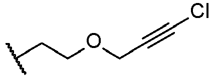 |
| **V-75** | H | 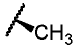 | 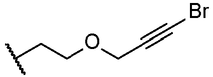 |
| **V-76** | H | 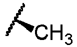 | 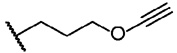 |
| **V-77** | H | 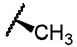 | 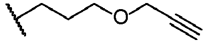 |
| **V-78** | H | 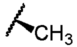 | 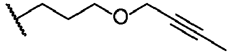 |
| **V-79** | H | 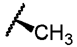 | 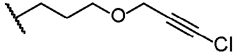 |
| **V-80** | H | 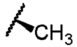 | 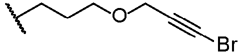 |
| **V-81** | H | 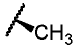 | 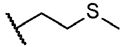 |
| **V-82** | H | 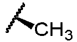 | 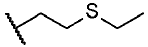 |
| **V-83** | H | 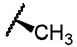 | 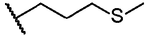 |
| **V-84** | H | 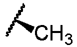 | 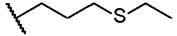 |
| **V-85** | H | 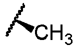 | 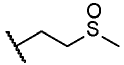 |
| **V-86** | H | 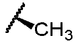 | 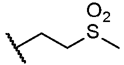 |
| **V-87** | H | 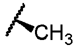 | 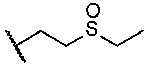 |
| **V-88** | H | 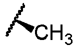 | 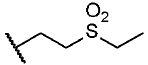 |
| **V-89** | H | 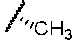 | 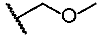 |
| **V-90** | H | 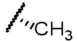 | 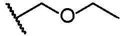 |
| **V-91** | H | 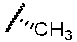 | 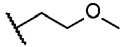 |
| **V-92** | H | 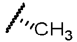 | 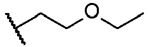 |
| **V-93** | H | 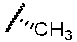 | 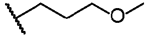 |
| **V-94** | H | 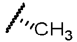 | 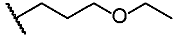 |
| **V-95** | H | 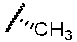 | 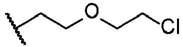 |
| **V-96** | H | 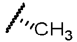 | 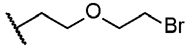 |
| **V-97** | H | 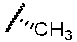 | 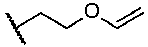 |
| **V-98** | H | 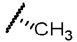 | 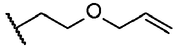 |
| **V-99** | H | 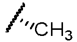 | 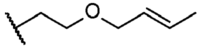 |
| **V-100** | H | 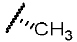 | 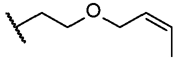 |
| **V-101** | H | 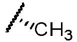 | 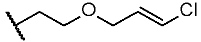 |
| **V-102** | H | 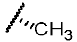 | 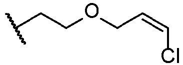 |
| **V-103** | H | 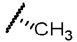 | 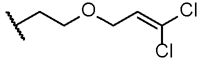 |
| **V-104** | H | 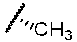 | 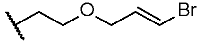 |
| **V-105** | H | 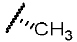 | 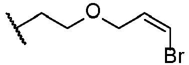 |
| **V-106** | H | 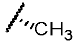 | 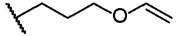 |
| **V-107** | H | 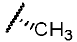 | 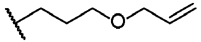 |
| **V-108** | H | 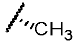 | 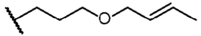 |
| **V-109** | H | 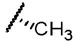 | 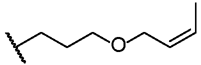 |
| **V-110** | H | 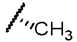 | 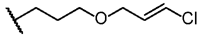 |
| **V-111** | H | 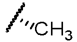 | 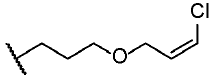 |
| **V-112** | H | 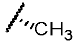 | 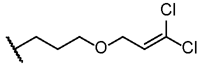 |
| **V-113** | H | 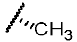 | 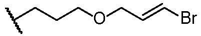 |
| **V-114** | H | 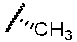 | 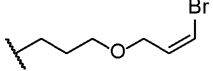 |
| **V-115** | H | 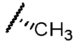 | 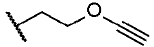 |
| **V-116** | H | 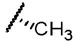 | 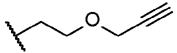 |
| **V-117** | H | 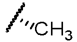 | 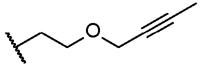 |
| **V-118** | H | 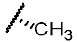 | 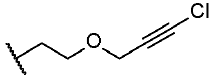 |
| **V-119** | H | 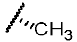 | 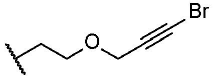 |
| **V-120** | H | 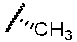 | 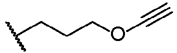 |
| **V-121** | H | 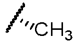 | 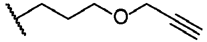 |
| **V-122** | H | 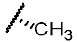 | 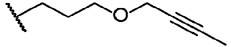 |
| **V-123** | H | 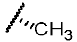 | 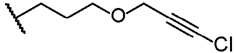 |
| **V-124** | H | 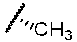 | 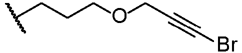 |
| **V-125** | H | 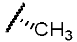 | 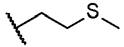 |
| **V-126** | H | 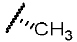 | 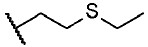 |
| **V-127** | H | 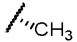 | 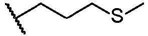 |
| **V-128** | H | 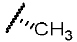 | 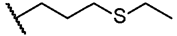 |
| **V-129** | H | 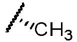 | 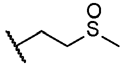 |
| **V-130** | H | 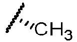 | 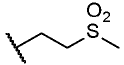 |
| **V-131** | H | 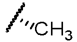 | 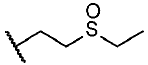 |
| **V-132** | H | 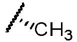 | 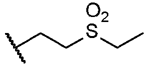 |
| **V-133** | H | CH₃ | 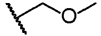 |
| **V-134** | H | CH₃ | 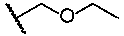 |
| **V-135** | H | CH₃ | 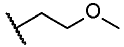 |
| **V-136** | H | CH₃ | 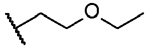 |
| **V-137** | H | CH₃ | 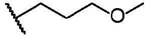 |
| **V-138** | H | CH₃ | 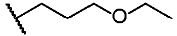 |
| **V-139** | H | CH₃ | 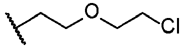 |
| **V-140** | H | CH₃ | 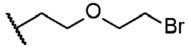 |
| **V-141** | H | CH₃ | 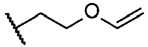 |
| **V-142** | H | CH₃ | 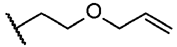 |
| **V-143** | H | CH₃ | 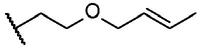 |
| **V-144** | H | CH₃ | 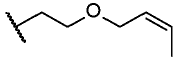 |
| **V-145** | H | CH₃ | 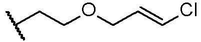 |
| **V-146** | H | CH₃ | 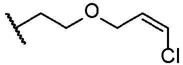 |
| **V-147** | H | CH₃ | 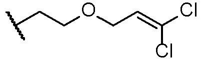 |
| **V-148** | H | CH₃ | 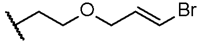 |
| **V-149** | H | CH₃ | 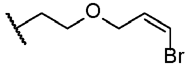 |
| **V-150** | H | CH₃ | 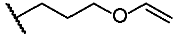 |
| **V-151** | H | CH₃ | 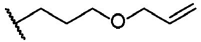 |
| **V-152** | H | CH₃ | 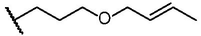 |
| **V-153** | H | CH₃ | 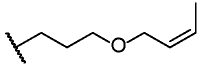 |
| **V-154** | H | CH₃ | 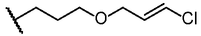 |
| **V-155** | H | CH₃ | 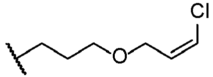 |
| **V-156** | H | CH₃ | 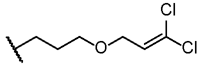 |
| **V-157** | H | CH₃ | 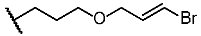 |
| **V-158** | H | CH₃ | 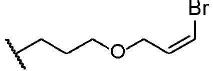 |
| **V-159** | H | CH₃ | 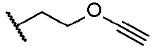 |
| **V-160** | H | CH₃ | 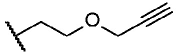 |
| **V-161** | H | CH₃ | 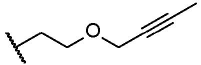 |
| **V-162** | H | CH₃ | 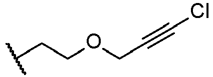 |
| **V-163** | H | CH₃ | 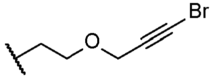 |
| **V-164** | H | CH₃ | 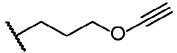 |
| **V-165** | H | CH₃ | 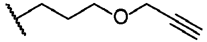 |
| **V-166** | H | CH₃ | 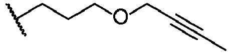 |
| **V-167** | H | CH₃ | 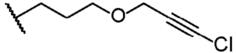 |
| **V-168** | H | CH₃ | 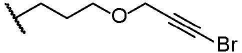 |
| **V-169** | H | CH₃ | 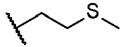 |
| **V-170** | H | CH₃ | 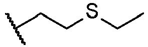 |
| **V-171** | H | CH₃ | 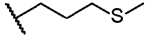 |
| **V-172** | H | CH₃ | 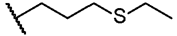 |
| **V-173** | H | CH₃ | 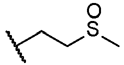 |
| **V-174** | H | CH₃ | 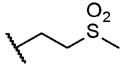 |
| **V-175** | H | CH₃ | 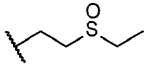 |
| **V-176** | H | CH₃ | 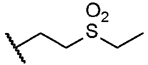 |
| **V-177** | CH₃ | CH₃ | 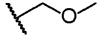 |
| **V-178** | CH₃ | CH₃ | 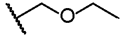 |
| **V-179** | CH₃ | CH₃ | 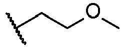 |
| **V-180** | CH₃ | CH₃ | 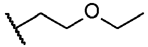 |
| **V-181** | CH₃ | CH₃ | 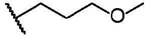 |
| **V-182** | CH₃ | CH₃ | 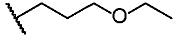 |
| **V-183** | CH₃ | CH₃ | 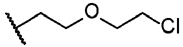 |
| **V-184** | CH₃ | CH₃ | 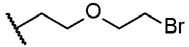 |
| **V-185** | CH₃ | CH₃ | 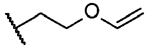 |
| **V-186** | CH₃ | CH₃ | 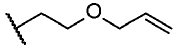 |
| **V-187** | CH₃ | CH₃ | 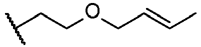 |
| **V-188** | CH₃ | CH₃ | 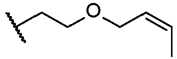 |
| **V-189** | CH₃ | CH₃ | 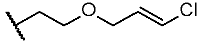 |
| **V-190** | CH₃ | CH₃ | 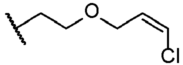 |
| **V-191** | CH₃ | CH₃ | 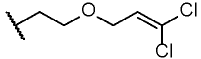 |
| **V-192** | CH₃ | CH₃ | 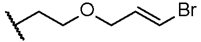 |
| **V-193** | CH₃ | CH₃ | 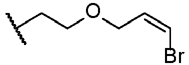 |
| **V-194** | CH₃ | CH₃ | 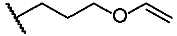 |
| **V-195** | CH₃ | CH₃ | 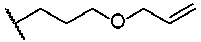 |
| **V-196** | CH₃ | CH₃ | 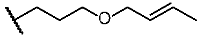 |
| **V-197** | CH₃ | CH₃ | 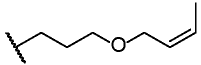 |
| **V-198** | CH₃ | CH₃ | 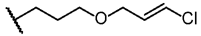 |
| **V-199** | CH₃ | CH₃ | 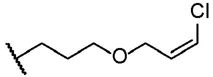 |
| **V-200** | CH₃ | CH₃ | 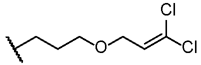 |
| **V-201** | CH₃ | CH₃ | 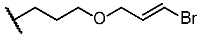 |
| **V-202** | CH₃ | CH₃ | 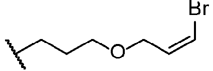 |
| **V-203** | CH₃ | CH₃ | 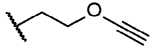 |
| **V-204** | CH₃ | CH₃ | 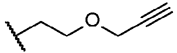 |
| **V-205** | CH₃ | CH₃ | 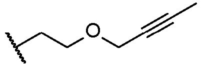 |
| **V-206** | CH₃ | CH₃ | 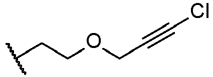 |
| **V-207** | CH₃ | CH₃ | 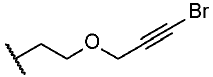 |
| **V-208** | CH₃ | CH₃ | 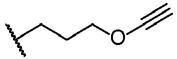 |
| **V-209** | CH₃ | CH₃ | 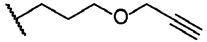 |
| **V-210** | CH₃ | CH₃ | 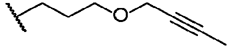 |
| **V-211** | CH₃ | CH₃ | 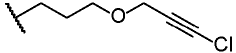 |
| **V-212** | CH₃ | CH₃ | 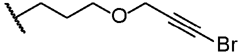 |
| **V-213** | CH₃ | CH₃ | 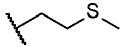 |
| **V-214** | CH₃ | CH₃ | 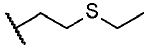 |
| **V-215** | CH₃ | CH₃ | 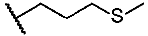 |
| **V-216** | CH₃ | CH₃ | 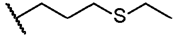 |
| **V-217** | CH₃ | CH₃ | 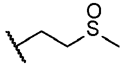 |
| **V-218** | CH₃ | CH₃ | 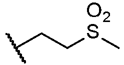 |
| **V-219** | CH₃ | CH₃ | 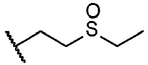 |
| **V-220** | CH₃ | CH₃ | 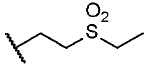 |
| **V-221** | -CH₂CH₂- | | 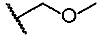 |
| **V-222** | -CH₂CH₂- | | 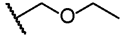 |
| **V-223** | -CH₂CH₂- | | 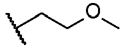 |
| **V-224** | -CH₂CH₂- | | 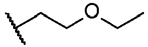 |
| **V-225** | -CH₂CH₂- | | 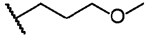 |
| **V-226** | -CH₂CH₂- | | 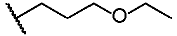 |
| **V-227** | -CH₂CH₂- | | 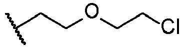 |
| **V-228** | -CH₂CH₂- | | 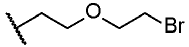 |
| **V-229** | -CH₂CH₂- | | 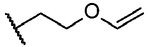 |
| **V-230** | -CH₂CH₂- | | 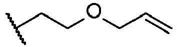 |
| **V-231** | -CH₂CH₂- | | 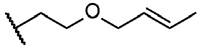 |
| **V-232** | -CH₂CH₂- | | 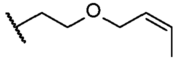 |
| **V-233** | -CH₂CH₂- | | 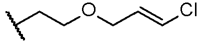 |
| **V-234** | -CH₂CH₂- | | 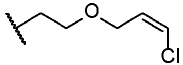 |
| **V-235** | -CH₂CH₂- | | 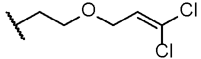 |
| **V-236** | -CH₂CH₂- | | 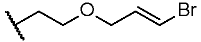 |
| **V-237** | -CH₂CH₂- | | 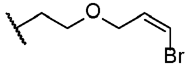 |
| **V-238** | -CH₂CH₂- | | 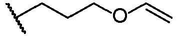 |
| **V-239** | -CH₂CH₂- | | 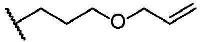 |
| | **V-240** | -CH₂CH₂- | 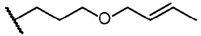 |
| | **V-241** | -CH₂CH₂- | 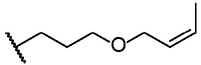 |
| | **V-242** | -CH₂CH₂- | 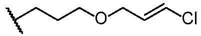 |
| | **V-243** | -CH₂CH₂- | 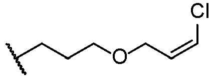 |
| | **V-244** | -CH₂CH₂- | 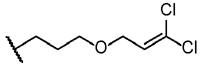 |
| | **V-245** | -CH₂CH₂- | 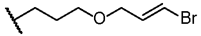 |
| | **V-246** | -CH₂CH₂- | 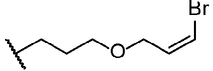 |
| | **V-247** | -CH₂CH₂- | 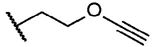 |
| | **V-248** | -CH₂CH₂- | 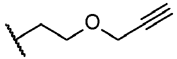 |
| | **V-249** | -CH₂CH₂- | 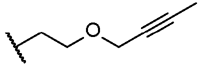 |
| | **V-250** | -CH₂CH₂- | 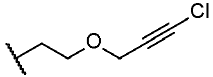 |
| | **V-251** | -CH₂CH₂- | 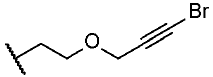 |
| | **V-252** | -CH₂CH₂- | 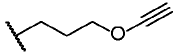 |
| | **V-253** | -CH₂CH₂- | 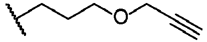 |
| | **V-254** | -CH₂CH₂- | 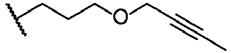 |
| | **V-255** | -CH₂CH₂- | 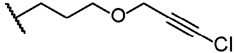 |
| | **V-256** | -CH₂CH₂- | 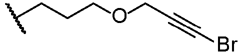 |
| | **V-257** | -CH₂CH₂- | 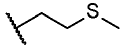 |
| | **V-258** | -CH₂CH₂- | 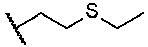 |
| | **V-259** | -CH₂CH₂- | 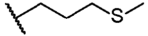 |
| | **V-260** | -CH₂CH₂- | 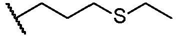 |
| | **V-261** | -CH₂CH₂- | 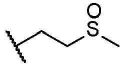 |
| | **V-262** | -CH₂CH₂- | 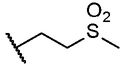 |
| | **V-263** | -CH₂CH₂- | 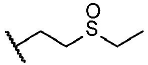 |
| **V-264** | -CH₂CH₂- | 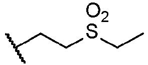 | |

The present invention also provides an aniline compound of formula (IV): 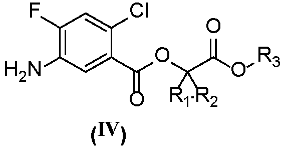
wherein R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

Preferably, in the aniline compound of formula (IV), R₁ and R₂ are each independently selected from hydrogen or methyl;
R₃ is selected from C_{1∼3} alkoxy-C_{1~3} alkyl, C_{1∼3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1∼3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

Some intermediate compounds of the present invention can be described using the specific compounds listed in Table 2, but the present invention is not limited to these compounds.

**Table 2 Structure of some compounds with general formula (IV)**

| NO. | R₁ | R₂ | R₃ |
|---|---|---|---|
| **IV-1** | H | H | 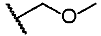 |
| **IV-2** | H | H | 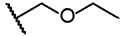 |
| **IV-3** | H | H | 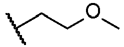 |
| **IV-4** | H | H | 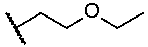 |
| **IV-5** | H | H | 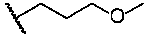 |
| **IV-6** | H | H | 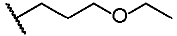 |
| **IV-7** | H | H | 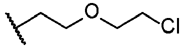 |
| **IV-8** | H | H | 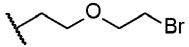 |
| **IV-9** | H | H | 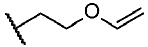 |
| **IV-10** | H | H | 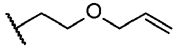 |
| **IV-11** | H | H | 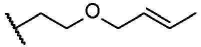 |
| **IV-12** | H | H | 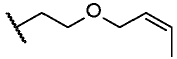 |
| **IV-13** | H | H | 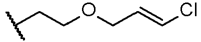 |
| **IV-14** | H | H | 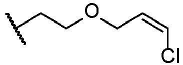 |
| **IV-15** | H | H | 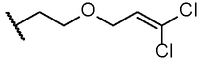 |
| **IV-16** | H | H | 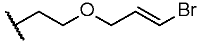 |
| **IV-17** | H | H | 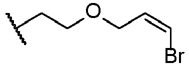 |
| **IV-18** | H | H | 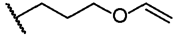 |
| **IV-19** | H | H | 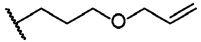 |
| **IV-20** | H | H | 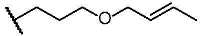 |
| **IV-21** | H | H | 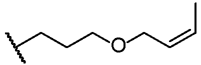 |
| **IV-22** | H | H | 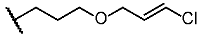 |
| **IV-23** | H | H | 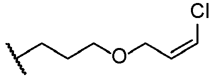 |
| **IV-24** | H | H | 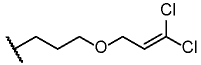 |
| **IV-25** | H | H | 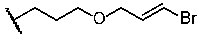 |
| **IV-26** | H | H | 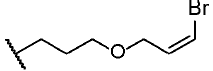 |
| **IV-27** | H | H | 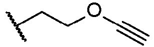 |
| **IV-28** | H | H | 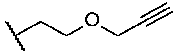 |
| **IV-29** | H | H | 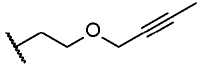 |
| **IV-30** | H | H | 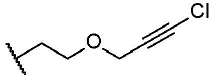 |
| **IV-31** | H | H | 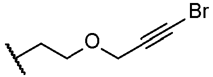 |
| **IV-32** | H | H | 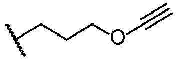 |
| **IV-33** | H | H | 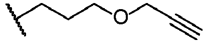 |
| **IV-34** | H | H | 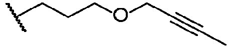 |
| **IV-35** | H | H | 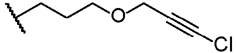 |
| **IV-36** | H | H | 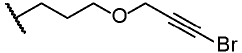 |
| **IV-37** | H | H | 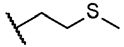 |
| **IV-38** | H | H | 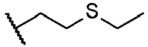 |
| **IV-39** | H | H | 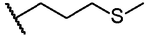 |
| **IV-40** | H | H | 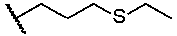 |
| **IV-41** | H | H | 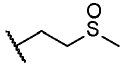 |
| **IV-42** | H | H | 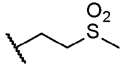 |
| **IV-43** | H | H | 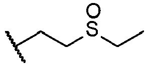 |
| **IV-44** | H | H | 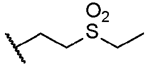 |
| **IV-45** | H | 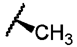 | 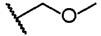 |
| **IV-46** | H | 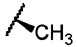 | 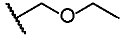 |
| **IV-47** | H | 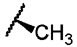 | 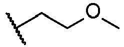 |
| **IV-48** | H | 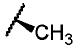 | 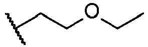 |
| **IV-49** | H | 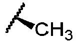 | 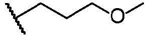 |
| **IV-50** | H | 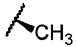 | 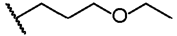 |
| **IV-51** | H | 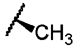 | 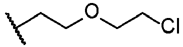 |
| **IV-52** | H | 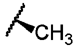 | 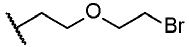 |
| **IV-53** | H | 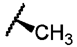 | 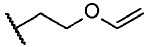 |
| **IV-54** | H | 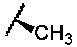 | 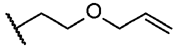 |
| **IV-55** | H | 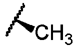 | 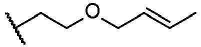 |
| **IV-56** | H | 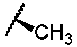 | 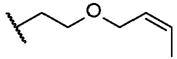 |
| **IV-57** | H | 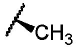 | 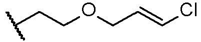 |
| **IV-58** | H | 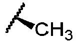 | 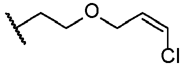 |
| **IV-59** | H | 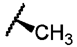 | 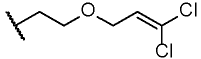 |
| **IV-60** | H | 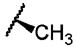 | 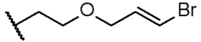 |
| **IV-61** | H | 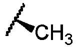 | 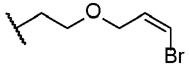 |
| **IV-62** | H | 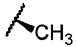 | 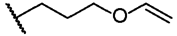 |
| **IV-63** | H | 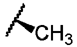 | 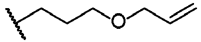 |
| **IV-64** | H | 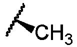 | 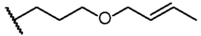 |
| **IV-65** | H | 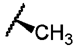 | 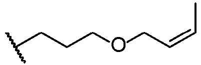 |
| **IV-66** | H | 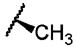 | 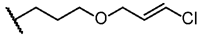 |
| **IV-67** | H | 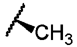 | 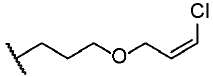 |
| **IV-68** | H | 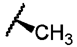 | 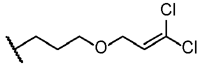 |
| **IV-69** | H | 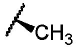 | 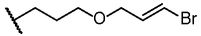 |
| **IV-70** | H | 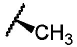 | 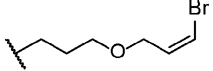 |
| **IV-71** | H | 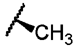 | 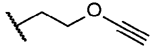 |
| **IV-72** | H | 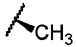 | 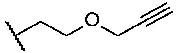 |
| **IV-73** | H | 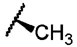 | 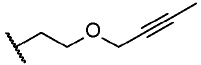 |
| **IV-74** | H | 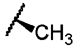 | 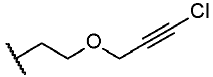 |
| **IV-75** | H | 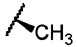 | 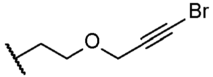 |
| **IV-76** | H | 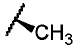 | 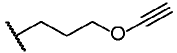 |
| **IV-77** | H | 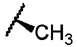 | 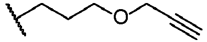 |
| **IV-78** | H | 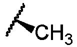 | 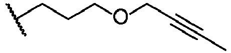 |
| **IV-79** | H | 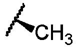 | 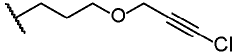 |
| **IV-80** | H | 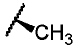 | 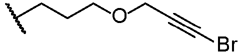 |
| **IV-81** | H | 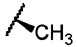 | 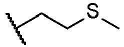 |
| **IV-82** | H | 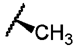 | 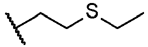 |
| **IV-83** | H | 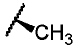 | 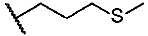 |
| **IV-84** | H | 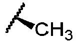 | 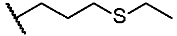 |
| **IV-85** | H | 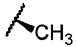 | 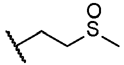 |
| **IV-86** | H | 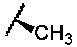 | 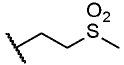 |
| **IV-87** | H | 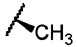 | 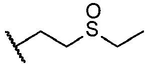 |
| **IV-88** | H | 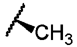 | 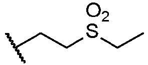 |
| **IV-89** | H | 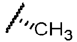 | 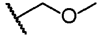 |
| **IV-90** | H | 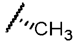 | 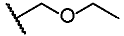 |
| **IV-91** | H | 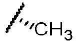 | 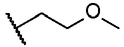 |
| **IV-92** | H | 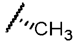 | 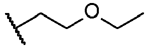 |
| **IV-93** | H | 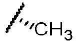 | 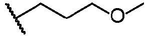 |
| **IV-94** | H | 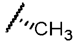 | 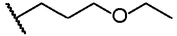 |
| **IV-95** | H | 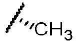 | 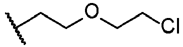 |
| **IV-96** | H | 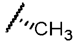 | 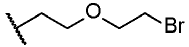 |
| **IV-97** | H | 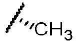 | 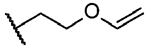 |
| **IV-98** | H | 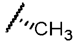 | 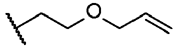 |
| **IV-99** | H | 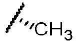 | 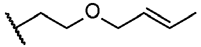 |
| **IV-100** | H | 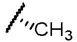 | 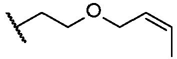 |
| **IV-101** | H | 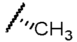 | 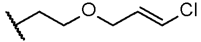 |
| **IV-102** | H | 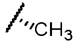 | 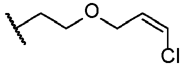 |
| **IV-103** | H | 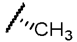 | 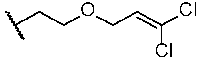 |
| **IV-104** | H | 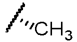 | 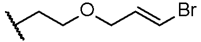 |
| **IV-105** | H | 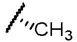 | 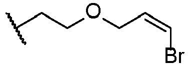 |
| **IV-106** | H | 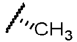 | 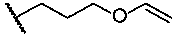 |
| **IV-107** | H | 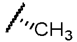 | 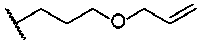 |
| **IV-108** | H | 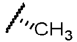 | 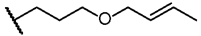 |
| **IV-109** | H | 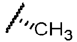 | 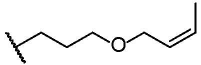 |
| **IV-110** | H | 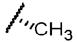 | 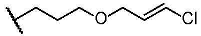 |
| **IV-111** | H | 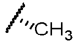 | 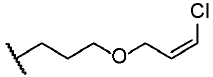 |
| **IV-112** | H | 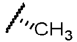 | 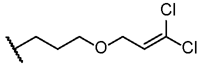 |
| **IV-113** | H | 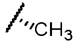 | 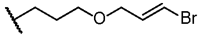 |
| **IV-114** | H | 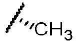 | 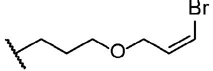 |
| **IV-115** | H | 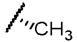 | 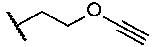 |
| **IV-116** | H | 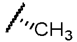 | 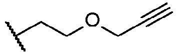 |
| **IV-117** | H | 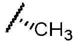 | 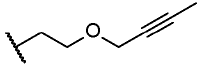 |
| **IV-118** | H | 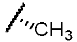 | 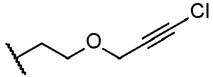 |
| **IV-119** | H | 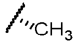 | 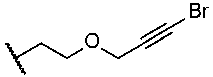 |
| **IV-120** | H | 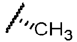 | 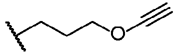 |
| **IV-121** | H | 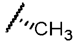 | 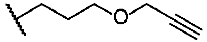 |
| **IV-122** | H | 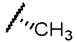 | 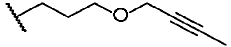 |
| **IV-123** | H | 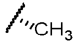 | 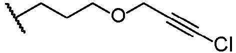 |
| **IV-124** | H | 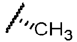 | 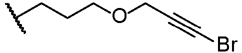 |
| **IV-125** | H | 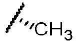 | 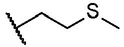 |
| **IV-126** | H | 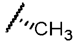 | 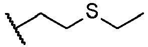 |
| **IV-127** | H | 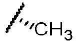 | 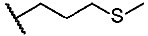 |
| **IV-128** | H | 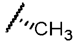 | 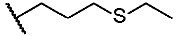 |
| **IV-129** | H | 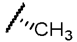 | 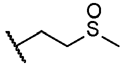 |
| **IV-130** | H | 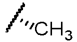 | 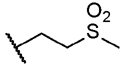 |
| **IV-131** | H | 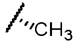 | 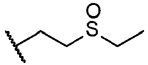 |
| **IV-132** | H | 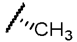 | 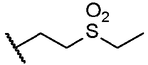 |
| **IV-133** | H | CH₃ | 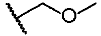 |
| **IV-134** | H | CH₃ | 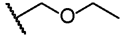 |
| **IV-135** | H | CH₃ | 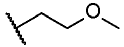 |
| **IV-136** | H | CH₃ | 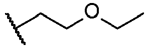 |
| **IV-137** | H | CH₃ | 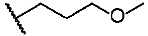 |
| **IV-138** | H | CH₃ | 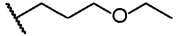 |
| **IV-139** | H | CH₃ | 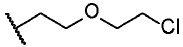 |
| **IV-140** | H | CH₃ | 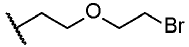 |
| **IV-141** | H | CH₃ | 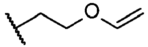 |
| **IV-142** | H | CH₃ | 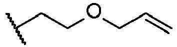 |
| **IV-143** | H | CH₃ | 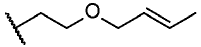 |
| **IV-144** | H | CH₃ | 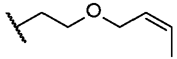 |
| **IV-145** | H | CH₃ | 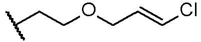 |
| **IV-146** | H | CH₃ | 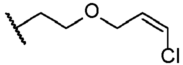 |
| **IV-147** | H | CH₃ | 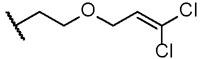 |
| **IV-148** | H | CH₃ | 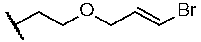 |
| **IV-149** | H | CH₃ | 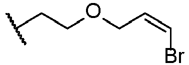 |
| **IV-150** | H | CH₃ | 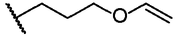 |
| **IV-151** | H | CH₃ | 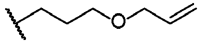 |
| **IV-152** | H | CH₃ | 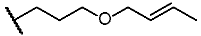 |
| **IV-153** | H | CH₃ | 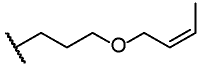 |
| **IV-154** | H | CH₃ | 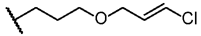 |
| **IV-155** | H | CH₃ | 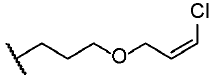 |
| **IV-156** | H | CH₃ | 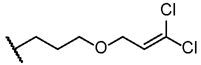 |
| **IV-157** | H | CH₃ | 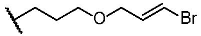 |
| **IV-158** | H | CH₃ | 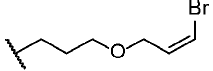 |
| **IV-159** | H | CH₃ | 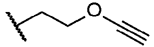 |
| **IV-160** | H | CH₃ | 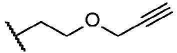 |
| **IV-161** | H | CH₃ | 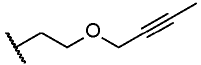 |
| **IV-162** | H | CH₃ | 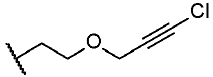 |
| **IV-163** | H | CH₃ | 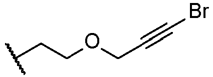 |
| **IV-164** | H | CH₃ | 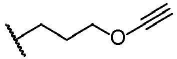 |
| **IV-165** | H | CH₃ | 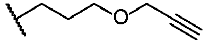 |
| **IV-166** | H | CH₃ | 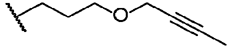 |
| **IV-167** | H | CH₃ | 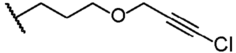 |
| **IV-168** | H | CH₃ | 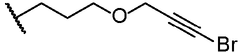 |
| **IV-169** | H | CH₃ | 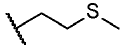 |
| **IV-170** | H | CH₃ | 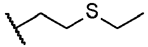 |
| **IV-171** | H | CH₃ | 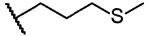 |
| **IV-172** | H | CH₃ | 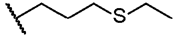 |
| **IV-173** | H | CH₃ | 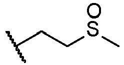 |
| **IV-174** | H | CH₃ | 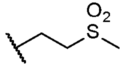 |
| **IV-175** | H | CH₃ | 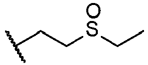 |
| **IV-176** | H | CH₃ | 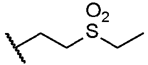 |
| **IV-177** | CH₃ | CH₃ | 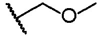 |
| **IV-178** | CH₃ | CH₃ | 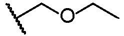 |
| **IV-179** | CH₃ | CH₃ | 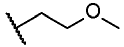 |
| **IV-180** | CH₃ | CH₃ | 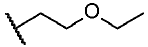 |
| **IV-181** | CH₃ | CH₃ | 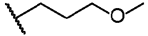 |
| **IV-182** | CH₃ | CH₃ | 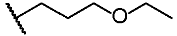 |
| **IV-183** | CH₃ | CH₃ | 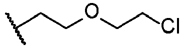 |
| **IV-184** | CH₃ | CH₃ | 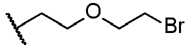 |
| **IV-185** | CH₃ | CH₃ | 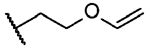 |
| **IV-186** | CH₃ | CH₃ | 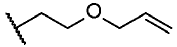 |
| **IV-187** | CH₃ | CH₃ | 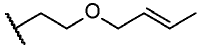 |
| **IV-188** | CH₃ | CH₃ | 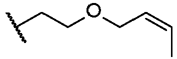 |
| **IV-189** | CH₃ | CH₃ | 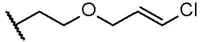 |
| **IV-190** | CH₃ | CH₃ | 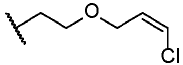 |
| **IV-191** | CH₃ | CH₃ | 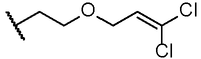 |
| **IV-192** | CH₃ | CH₃ | 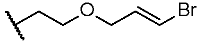 |
| **IV-193** | CH₃ | CH₃ | 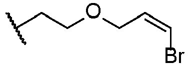 |
| **IV-194** | CH₃ | CH₃ | 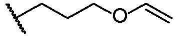 |
| **IV-195** | CH₃ | CH₃ | 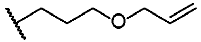 |
| **IV-196** | CH₃ | CH₃ | 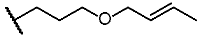 |
| **IV-197** | CH₃ | CH₃ | 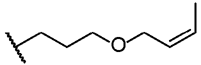 |
| **IV-198** | CH₃ | CH₃ | 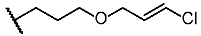 |
| **IV-199** | CH₃ | CH₃ | 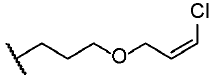 |
| **IV-200** | CH₃ | CH₃ | 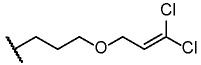 |
| **IV-201** | CH₃ | CH₃ | 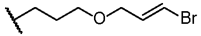 |
| **IV-202** | CH₃ | CH₃ | 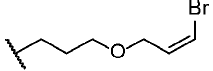 |
| **IV-203** | CH₃ | CH₃ | 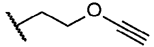 |
| **IV-204** | CH₃ | CH₃ | 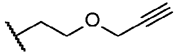 |
| **IV-205** | CH₃ | CH₃ | 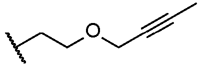 |
| **IV-206** | CH₃ | CH₃ | 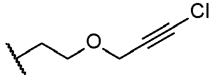 |
| **IV-207** | CH₃ | CH₃ | 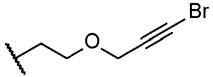 |
| **IV-208** | CH₃ | CH₃ | 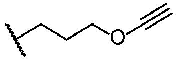 |
| **IV-209** | CH₃ | CH₃ | 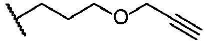 |
| **IV-210** | CH₃ | CH₃ | 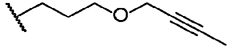 |
| **IV-211** | CH₃ | CH₃ | 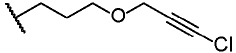 |
| **IV-212** | CH₃ | CH₃ | 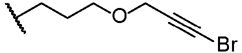 |
| **IV-213** | CH₃ | CH₃ | 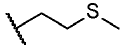 |
| **IV-214** | CH₃ | CH₃ | 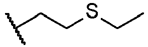 |
| **IV-215** | CH₃ | CH₃ | 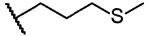 |
| **IV-216** | CH₃ | CH₃ | 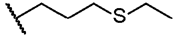 |
| **IV-217** | CH₃ | CH₃ | 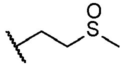 |
| **IV-218** | CH₃ | CH₃ | 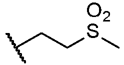 |
| **IV-219** | CH₃ | CH₃ | 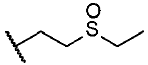 |
| **IV-220** | CH₃ | CH₃ | 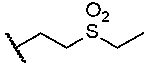 |
| **IV-221** | -CH₂CH₂- | | 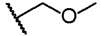 |
| **IV-222** | -CH₂CH₂- | | 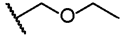 |
| **IV-223** | -CH₂CH₂- | 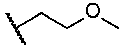 | |
| **IV-224** | -CH₂CH₂- | 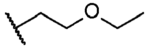 | |
| **IV-225** | -CH₂CH₂- | 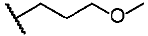 | |
| **IV-226** | -CH₂CH₂- | 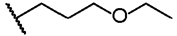 | |
| **IV-227** | -CH₂CH₂- | 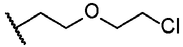 | |
| **IV-228** | -CH₂CH₂- | 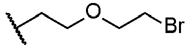 | |
| **IV-229** | -CH₂CH₂- | 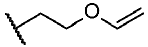 | |
| **IV-230** | -CH₂CH₂- | 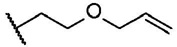 | |
| **IV-231** | -CH₂CH₂- | 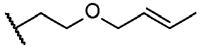 | |
| **IV-232** | -CH₂CH₂- | 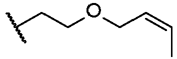 | |
| **IV-233** | -CH₂CH₂- | 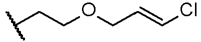 | |
| **IV-234** | -CH₂CH₂- | 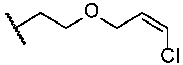 | |
| **IV-235** | -CH₂CH₂- | 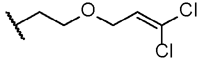 | |
| **IV-236** | -CH₂CH₂- | 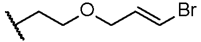 | |
| **IV-237** | -CH₂CH₂- | 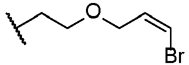 | |
| **IV-238** | -CH₂CH₂- | 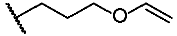 | |
| **IV-239** | -CH₂CH₂- | 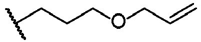 | |
| **IV-240** | -CH₂CH₂- | 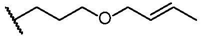 | |
| **IV-241** | -CH₂CH₂- | 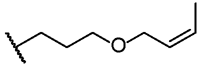 | |
| **IV-242** | -CH₂CH₂- | 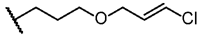 | |
| **IV-243** | -CH₂CH₂- | 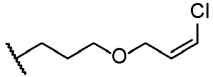 | |
| **IV-244** | -CH₂CH₂- | 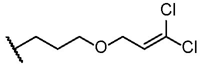 | |
| **IV-245** | -CH₂CH₂- | 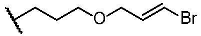 | |
| **IV-246** | -CH₂CH₂- | 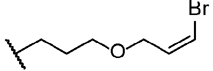 | |
| **IV-247** | -CH₂CH₂- | 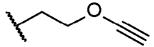 | |
| **IV-248** | -CH₂CH₂- | 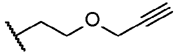 | |
| **IV-249** | -CH₂CH₂- | 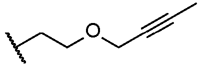 | |
| **IV-250** | -CH₂CH₂- | 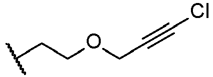 | |
| **IV-251** | -CH₂CH₂- | 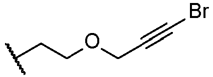 | |
| **IV-252** | -CH₂CH₂- | 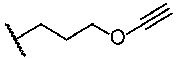 | |
| **IV-253** | -CH₂CH₂- | 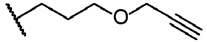 | |
| **IV-254** | -CH₂CH₂- | 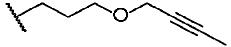 | |
| **IV-255** | -CH₂CH₂- | 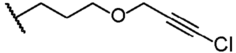 | |
| **IV-256** | -CH₂CH₂- | 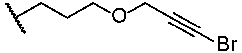 | |
| **IV-257** | -CH₂CH₂- | 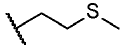 | |
| **IV-258** | -CH₂CH₂- | 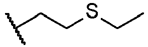 | |
| **IV-259** | -CH₂CH₂- | 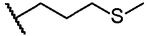 | |
| **IV-260** | -CH₂CH₂- | 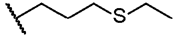 | |
| **IV-261** | -CH₂CH₂- | 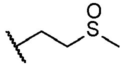 | |
| **IV-262** | -CH₂CH₂- | 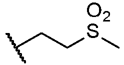 | |
| **IV-263** | -CH₂CH₂- | 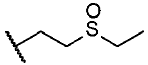 | |
| **IV-264** | -CH₂CH₂- | 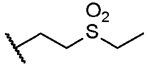 | |

The present invention also provides a nitrobenzene compound of formula (III): 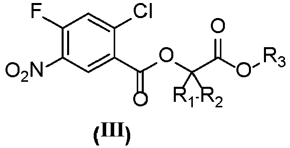
wherein R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

Preferably, in the nitrobenzene compound of formula **(III),** R₁ and R₂ are each independently selected from hydrogen or methyl;
R₃ is selected from C_{1∼3} alkoxy-C_{1~3} alkyl, C_{1∼3} halogenated alkoxy-C_{1~3} alkyl, C_{2~6} enyloxy-C_{1~3} alkyl, C_{2~6} halogenated enyloxy-C_{1∼3} alkyl, C_{2~6} ynyloxy-C_{1∼3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

Some intermediate compounds of the present invention can be described using the specific compounds listed in Table 3, but the present invention is not limited to these compounds.

**Table 3 Structure of some compounds with general formula (III)**

| NO. | R₁ | R₂ | R₃ |
|---|---|---|---|
| **III-1** | H | H | 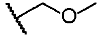 |
| **III-2** | H | H | 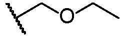 |
| **III-3** | H | H | 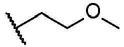 |
| **III-4** | H | H | 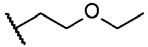 |
| **III-5** | H | H | 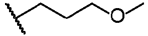 |
| **III-6** | H | H | 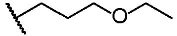 |
| **III-7** | H | H | 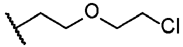 |
| **III-8** | H | H | 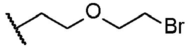 |
| **III-9** | H | H | 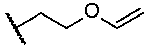 |
| **III-10** | H | H | 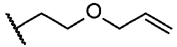 |
| **III-11** | H | H | 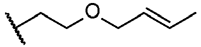 |
| **III-12** | H | H | 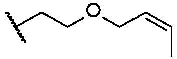 |
| **III-13** | H | H | 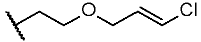 |
| **III-14** | H | H | 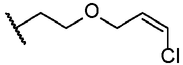 |
| **III-15** | H | H | 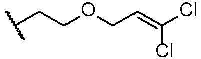 |
| **III-16** | H | H | 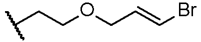 |
| **III-17** | H | H | 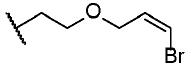 |
| **III-18** | H | H | 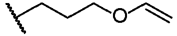 |
| **III-19** | H | H | 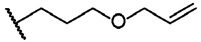 |
| **III-20** | H | H | 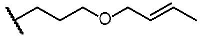 |
| **III-21** | H | H | 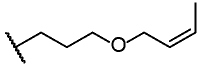 |
| **III-22** | H | H | 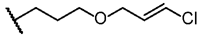 |
| **III-23** | H | H | 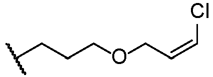 |
| **III-24** | H | H | 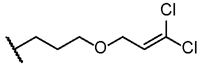 |
| **III-25** | H | H | 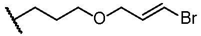 |
| **III-26** | H | H | 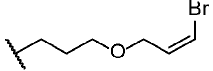 |
| **III-27** | H | H | 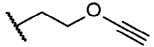 |
| **III-28** | H | H | 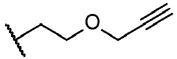 |
| **III-29** | H | H | 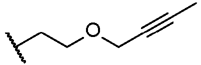 |
| **III-30** | H | H | 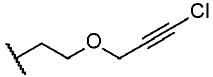 |
| **III-31** | H | H | 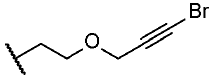 |
| **III-32** | H | H | 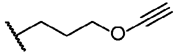 |
| **III-33** | H | H | 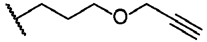 |
| **III-34** | H | H | 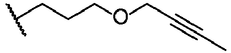 |
| **III-35** | H | H | 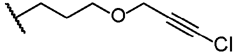 |
| **III-36** | H | H | 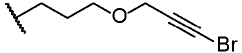 |
| **III-37** | H | H | 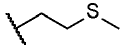 |
| **III-38** | H | H | 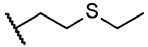 |
| **III-39** | H | H | 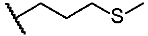 |
| **III-40** | H | H | 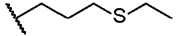 |
| **III-41** | H | H | 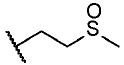 |
| **III-42** | H | H | 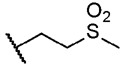 |
| **III-43** | H | H | 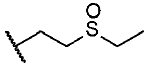 |
| **III-44** | H | H | 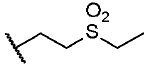 |
| **III-45** | H | 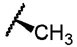 | 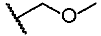 |
| **III-46** | H | 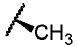 | 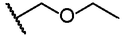 |
| **III-47** | H | 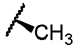 | 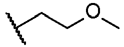 |
| **III-48** | H | 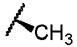 | 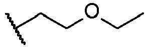 |
| **III-49** | H | 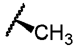 | 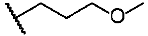 |
| **III-50** | H | 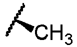 | 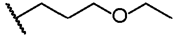 |
| **III-51** | H | 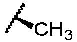 | 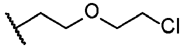 |
| **III-52** | H | 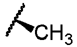 | 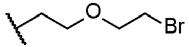 |
| **III-53** | H | 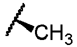 | 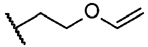 |
| **III-54** | H | 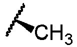 | 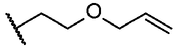 |
| **III-55** | H | 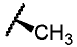 | 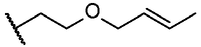 |
| **III-56** | H | 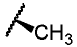 | 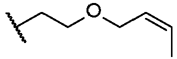 |
| **III-57** | H | 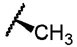 | 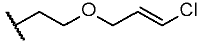 |
| **III-58** | H | 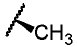 | 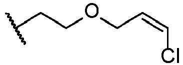 |
| **III-59** | H | 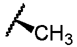 | 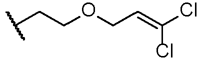 |
| **III-60** | H | 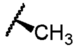 | 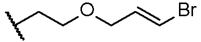 |
| **III-61** | H | 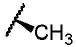 | 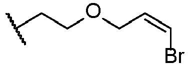 |
| **III-62** | H | 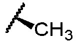 | 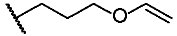 |
| **III-63** | H | 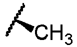 | 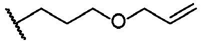 |
| **III-64** | H | 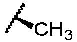 | 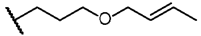 |
| **III-65** | H | 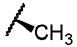 | 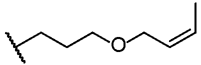 |
| **III-66** | H | 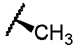 | 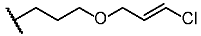 |
| **III-67** | H | 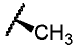 | 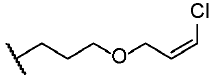 |
| **III-68** | H | 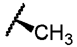 | 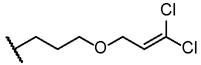 |
| **III-69** | H | 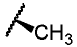 | 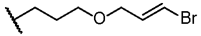 |
| **III-70** | H | 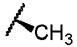 | 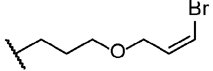 |
| **III-71** | H | 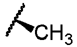 | 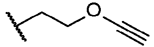 |
| **III-72** | H | 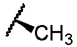 | 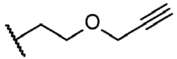 |
| **III-73** | H | 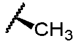 | 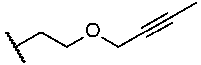 |
| **III-74** | H | 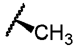 | 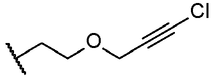 |
| **III-75** | H | 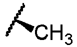 | 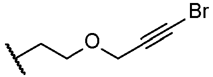 |
| **III-76** | H | 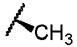 | 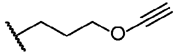 |
| **III-77** | H | 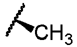 | 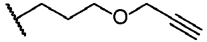 |
| **III-78** | H | 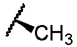 | 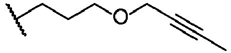 |
| **III-79** | H | 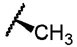 | 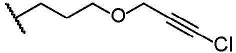 |
| **III-80** | H | 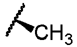 | 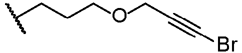 |
| **III-81** | H | 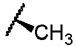 | 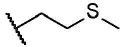 |
| **III-82** | H | 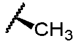 | 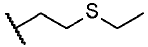 |
| **III-83** | H | 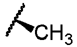 | 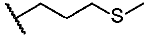 |
| **III-84** | H | 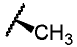 | 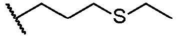 |
| **III-85** | H | 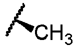 | 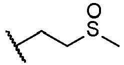 |
| **III-86** | H | 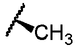 | 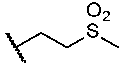 |
| **III-87** | H | 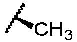 | 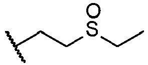 |
| **III-88** | H | 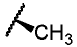 | 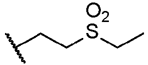 |
| **III-89** | H | 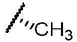 | 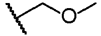 |
| **III-90** | H | 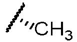 | 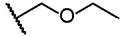 |
| **III-91** | H | 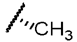 | 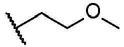 |
| **III-92** | H | 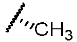 | 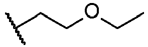 |
| **III-93** | H | 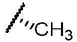 | 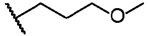 |
| **III-94** | H | 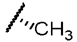 | 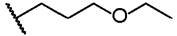 |
| III-95 | H | 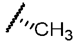 | 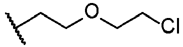 |
| III-96 | H | 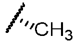 | 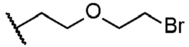 |
| III-97 | H | 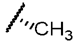 | 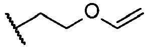 |
| III-98 | H | 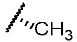 | 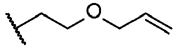 |
| **III-99** | H | 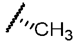 | 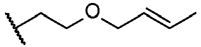 |
| **III-100** | H | 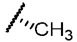 | 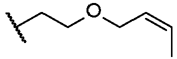 |
| **III-101** | H | 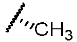 | 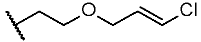 |
| **III-102** | H | 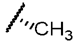 | 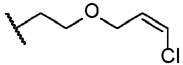 |
| **III-103** | H | 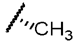 | 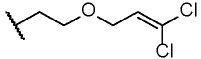 |
| **III-104** | H | 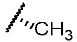 | 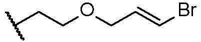 |
| **III-105** | H | 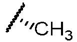 | 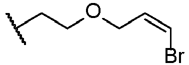 |
| **III-106** | H | 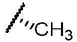 | 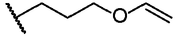 |
| **III-107** | H | 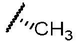 | 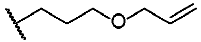 |
| **III-108** | H | 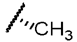 | 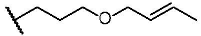 |
| **III-109** | H | 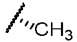 | 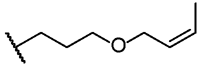 |
| **III-110** | H | 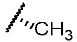 | 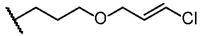 |
| **III-111** | H | 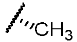 | 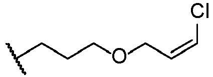 |
| **III-112** | H | 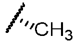 | 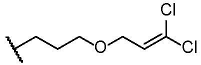 |
| **III-113** | H | 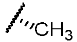 | 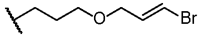 |
| **III-114** | H | 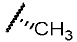 | 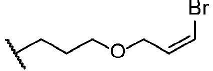 |
| **III-115** | H | 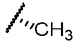 | 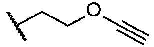 |
| **III-116** | H | 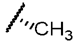 | 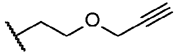 |
| **III-117** | H | 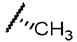 | 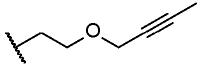 |
| **III-118** | H | 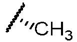 | 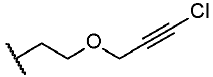 |
| **III-119** | H | 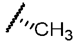 | 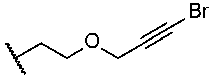 |
| **III-120** | H | 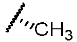 | 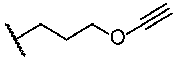 |
| **III-121** | H | 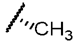 | 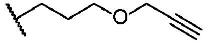 |
| **III-122** | H | 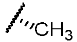 | 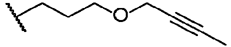 |
| **III-123** | H | 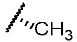 | 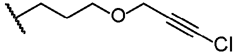 |
| **III-124** | H | 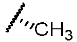 | 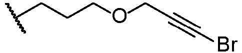 |
| **III-125** | H | 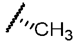 | 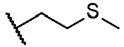 |
| **III-126** | H | 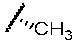 | 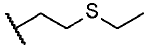 |
| **III-127** | H | 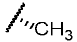 | 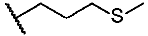 |
| **III-128** | H | 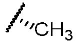 | 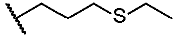 |
| **III-129** | H | 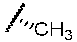 | 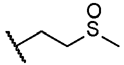 |
| **III-130** | H | 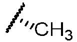 | 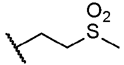 |
| **III-131** | H | 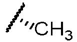 | 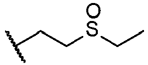 |
| **III-132** | H | 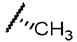 | 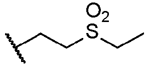 |
| **III-133** | H | CH₃ | 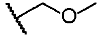 |
| **III-134** | H | CH₃ | 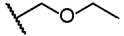 |
| **III-135** | H | CH₃ | 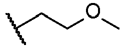 |
| **III-136** | H | CH₃ | 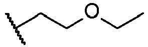 |
| **III-137** | H | CH₃ | 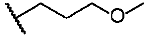 |
| **III-138** | H | CH₃ | 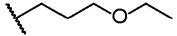 |
| **III-139** | H | CH₃ | 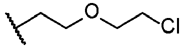 |
| **III-140** | H | CH₃ | 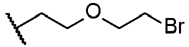 |
| **III-141** | H | CH₃ | 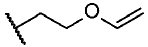 |
| **III-142** | H | CH₃ | 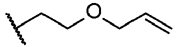 |
| **III-143** | H | CH₃ | 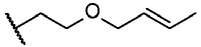 |
| **III-144** | H | CH₃ | 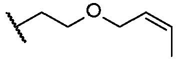 |
| **III-145** | H | CH₃ | 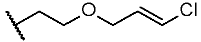 |
| **III-146** | H | CH₃ | 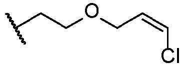 |
| **III-147** | H | CH₃ | 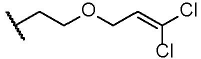 |
| **III-148** | H | CH₃ | 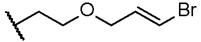 |
| **III-149** | H | CH₃ | 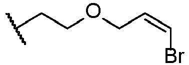 |
| **III-150** | H | CH₃ | 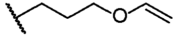 |
| **III-151** | H | CH₃ | 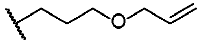 |
| **III-152** | H | CH₃ | 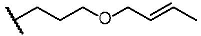 |
| **III-153** | H | CH₃ | 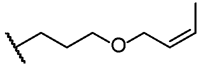 |
| **III-154** | H | CH₃ | 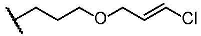 |
| **III-155** | H | CH₃ | 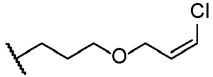 |
| **III-156** | H | CH₃ | 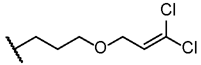 |
| **III-157** | H | CH₃ | 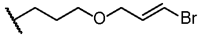 |
| **III-158** | H | CH₃ | 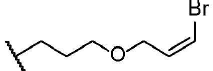 |
| **III-159** | H | CH₃ | 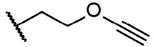 |
| **III-160** | H | CH₃ | 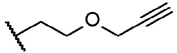 |
| **III-161** | H | CH₃ | 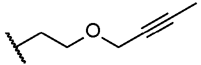 |
| **III-162** | H | CH₃ | 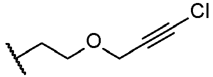 |
| **III-163** | H | CH₃ | 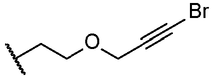 |
| **III-164** | H | CH₃ | 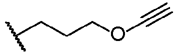 |
| **III-165** | H | CH₃ | 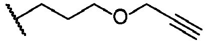 |
| **III-166** | H | CH₃ | 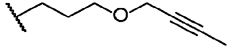 |
| **III-167** | H | CH₃ | 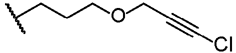 |
| **III-168** | H | CH₃ | 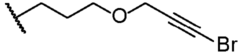 |
| **III-169** | H | CH₃ | 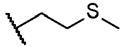 |
| **III-170** | H | CH₃ | 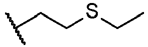 |
| **III-171** | H | CH₃ | 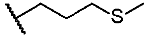 |
| **III-172** | H | CH₃ | 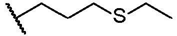 |
| **III-173** | H | CH₃ | 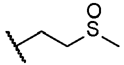 |
| **III-174** | H | CH₃ | 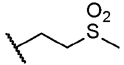 |
| **III-175** | H | CH₃ | 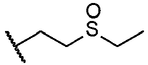 |
| **III-176** | H | CH₃ | 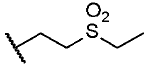 |
| **III-177** | CH₃ | CH₃ | 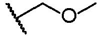 |
| **III-178** | CH₃ | CH₃ | 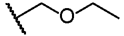 |
| **III-179** | CH₃ | CH₃ | 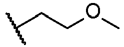 |
| **III-180** | CH₃ | CH₃ | 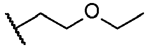 |
| **III-181** | CH₃ | CH₃ | 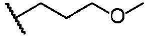 |
| **III-182** | CH₃ | CH₃ | 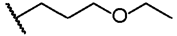 |
| **III-183** | CH₃ | CH₃ | 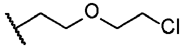 |
| **III-184** | CH₃ | CH₃ | 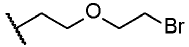 |
| **III-185** | CH₃ | CH₃ | 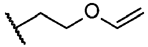 |
| **III-186** | CH₃ | CH₃ | 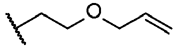 |
| **III-187** | CH₃ | CH₃ | 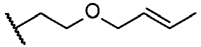 |
| **III-188** | CH₃ | CH₃ | 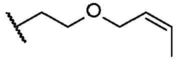 |
| **III-189** | CH₃ | CH₃ | 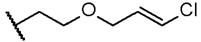 |
| **III-190** | CH₃ | CH₃ | 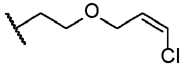 |
| **III-191** | CH₃ | CH₃ | 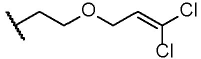 |
| **III-192** | CH₃ | CH₃ | 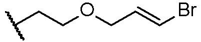 |
| **III-193** | CH₃ | CH₃ | 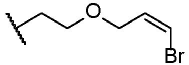 |
| **III-194** | CH₃ | CH₃ | 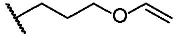 |
| **III-195** | CH₃ | CH₃ | 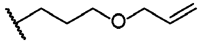 |
| **III-196** | CH₃ | CH₃ | 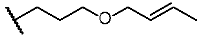 |
| **III-197** | CH₃ | CH₃ | 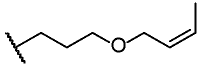 |
| **III-198** | CH₃ | CH₃ | 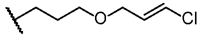 |
| **III-199** | CH₃ | CH₃ | 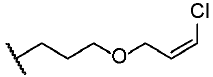 |
| **III-200** | CH₃ | CH₃ | 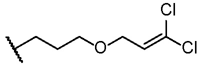 |
| **III-201** | CH₃ | CH₃ | 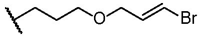 |
| **III-202** | CH₃ | CH₃ | 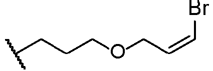 |
| **III-203** | CH₃ | CH₃ | 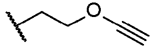 |
| **III-204** | CH₃ | CH₃ | 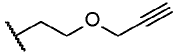 |
| **III-205** | CH₃ | CH₃ | 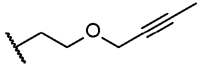 |
| **III-206** | CH₃ | CH₃ | 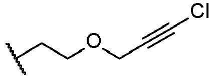 |
| **III-207** | CH₃ | CH₃ | 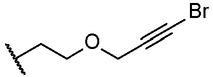 |
| **III-208** | CH₃ | CH₃ | 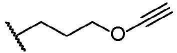 |
| **III-209** | CH₃ | CH₃ | 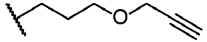 |
| **III-210** | CH₃ | CH₃ | 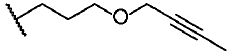 |
| **III-211** | CH₃ | CH₃ | 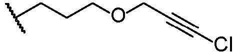 |
| **III-212** | CH₃ | CH₃ | 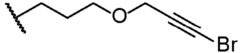 |
| **III-213** | CH₃ | CH₃ | 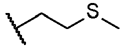 |
| **III-214** | CH₃ | CH₃ | 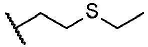 |
| **III-215** | CH₃ | CH₃ | 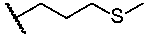 |
| **III-216** | CH₃ | CH₃ | 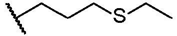 |
| **III-217** | CH₃ | CH₃ | 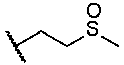 |
| **III-218** | CH₃ | CH₃ | 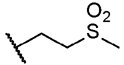 |
| **III-219** | CH₃ | CH₃ | 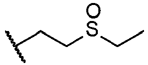 |
| **III-220** | CH₃ | CH₃ | 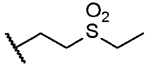 |
| **III-221** | -CH₂CH₂- | | 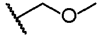 |
| **III-222** | -CH₂CH₂- | | 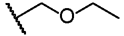 |
| **III-223** | -CH₂CH₂- | | 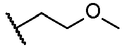 |
| **III-224** | -CH₂CH₂- | | 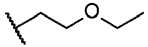 |
| **III-225** | -CH₂CH₂- | | 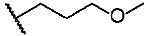 |
| **III-226** | -CH₂CH₂- | | 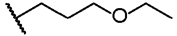 |
| **III-227** | -CH₂CH₂- | | 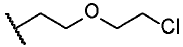 |
| **III-228** | -CH₂CH₂- | | 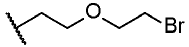 |
| **III-229** | -CH₂CH₂- | | 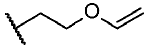 |
| **III-230** | -CH₂CH₂- | | 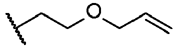 |
| **III-231** | -CH₂CH₂- | | 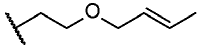 |
| **III-232** | -CH₂CH₂- | | 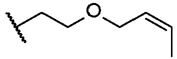 |
| **III-233** | -CH₂CH₂- | | 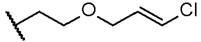 |
| **III-234** | -CH₂CH₂- | | 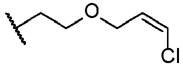 |
| **III-235** | -CH₂CH₂- | 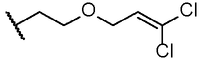 | |
| **III-236** | -CH₂CH₂- | 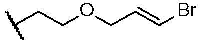 | |
| **III-237** | -CH₂CH₂- | 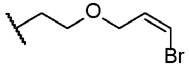 | |
| **III-238** | -CH₂CH₂- | 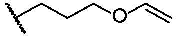 | |
| **III-239** | -CH₂CH₂- | 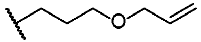 | |
| **III-240** | -CH₂CH₂- | 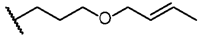 | |
| **III-241** | -CH₂CH₂- | 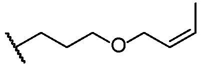 | |
| **III-242** | -CH₂CH₂- | 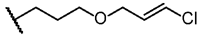 | |
| **III-243** | -CH₂CH₂- | 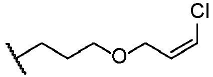 | |
| **III-244** | -CH₂CH₂- | 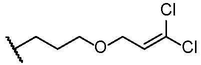 | |
| **III-245** | -CH₂CH₂- | 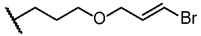 | |
| **III-246** | -CH₂CH₂- | 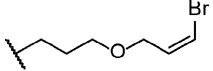 | |
| **III-247** | -CH₂CH₂- | 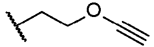 | |
| **III-248** | -CH₂CH₂- | 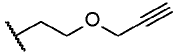 | |
| **III-249** | -CH₂CH₂- | 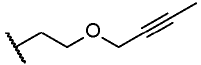 | |
| **III-250** | -CH₂CH₂- | 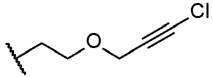 | |
| **III-251** | -CH₂CH₂- | 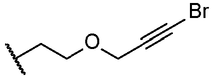 | |
| **III-252** | -CH₂CH₂- | 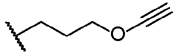 | |
| **III-253** | -CH₂CH₂- | 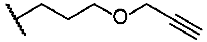 | |
| **III-254** | -CH₂CH₂- | 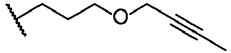 | |
| **III-255** | -CH₂CH₂- | 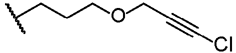 | |
| **III-256** | -CH₂CH₂- | 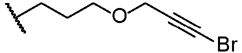 | |
| **III-257** | -CH₂CH₂- | 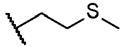 | |
| **III-258** | -CH₂CH₂- | 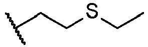 | |
| **III-259** | -CH₂CH₂- | 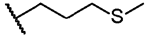 | |
| **III-260** | -CH₂CH₂- | 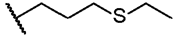 | |
| **III-261** | -CH₂CH₂- | 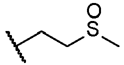 | |
| **III-262** | -CH₂CH₂- | 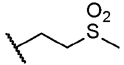 | |
| **III-263** | -CH₂CH₂- | 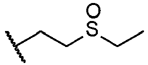 | |
| **III-264** | -CH₂CH₂- | 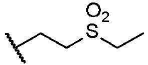 | |

The present invention also provides use of a uracil compound containing a carboxylate fragment of formula (V) in the preparation of 2-chloro-4-fluoro-5-(3-methyl-2,6-dioxo-4-(trifluoromethyl)-3,6-dihydropyrimidin-1(2H)-yl) benzoate compound of formula (I).

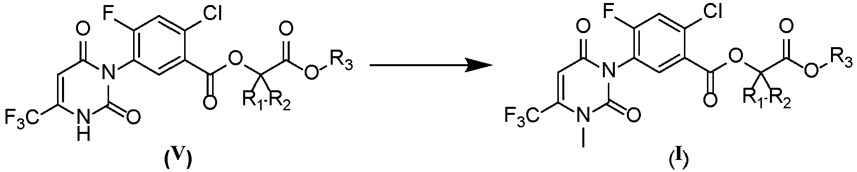

In the scheme, R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

The present invention also provides use of an aniline compound containing a carboxylate fragment of formula (IV) in the preparation of a uracil compound containing a carboxylate fragment of formula (V).

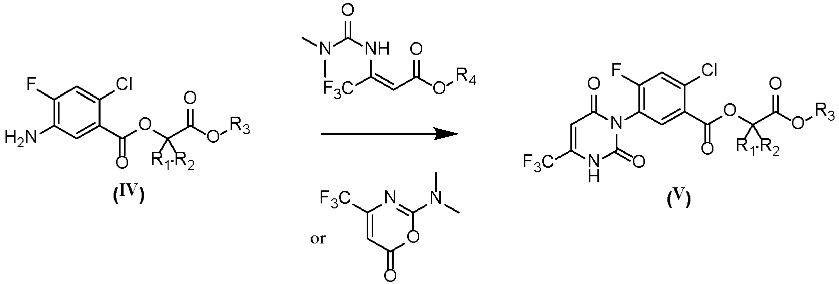

In the scheme, R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

The present invention also provides use of a nitrobenzene compound containing a carboxylate fragment of formula (III) in the preparation of an aniline compound containing a carboxylate fragment of formula (IV).

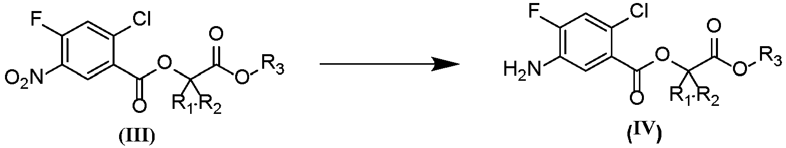

In the scheme, R₁ and R₂ are each independently selected from hydrogen or methyl; or R₁ and R₂ together with the carbon atom to which they are attached form a three-membered carbocycle;
R₃ is selected from C_{1∼3} alkoxy-C_{1∼3} alkyl, C_{1∼3} halogenated alkoxy-C_{1∼3} alkyl, C_{2~6} enyloxy-C_{1~3} alkyl, C_{2~6} halogenated enyloxy-C_{1~3} alkyl, C_{2~6} ynyloxy-C_{1~3} alkyl, C_{2~6} halogenated ynyloxy-C_{1~3} alkyl, or C_{1∼3} alkyl S(O)ₙ C_{1∼3} alkyl, n is 0, 1, or 2;
when R₁ is selected from hydrogen and R₂ is selected from methyl, the chiral carbon atom connected thereto is selected from either an R configuration or an S configuration, or a mixture of the two.

In the definition of general formula compounds given above, the terms used are gathered and generally defined as follows.

Halogen refers to fluorine, chlorine, bromine, or iodine. Alkyl refers to linear or branched alkyl, such as methyl, ethyl, propyl, isopropyl, n-butyl, tert-butyl or sec-butyl and isomer thereof. Alkenyl refers to linear or branched alkenyl, such as ethenyl, 1-propenyl, 2-propenyl, and different isomers of butenyl, pentenyl, and hexenyl. Alkenyl also includes polyene group, such as 1,2-prodienyl and 2,4-hexadienyl. Alkynyl refers to linear or branched alkynyl, such as ethynyl, propynyl, and different isomers of butynyl, pentynyl and hexynyl. Alkynyl also includes polyyne, such as 2,4-hexadiyne. Cycloalkyl refers to a substituted or unsubstituted cyclic alkyl, such as cyclobutyl or cyclopenty, and substituent groups such as methyl, halogen, cyano, etc. Cycloalkyl alkyl refers to a substituted or unsubstituted alkyl with a cyclic alkyl, such as cyclopropylmethyl, cyclobutylmethyl, and substituent groups such as methyl, halogen, cyano, etc. Halogenated alkyl refers linear or branched alkyl on which the hydrogen atoms can be partially or completely replaced by halogen atoms, such as chloropropyl, bromopropyl, etc. Alkoxyalkyl refers to alkyl-O-alkyl, such as CH₃OCH₂-. Halogenated alkoxyalkyl refers to alkyl-O-alkyl, on which the hydrogen atoms can be partially or completely replaced by halogen atoms, such as ClCH₂OCH₂-. Enyloxy alkyl refers to alkenyl-O-alkyl, for example, CH₂=CHCH₂OCH₂CH₂-. Halogenated enyloxy alkyl refers to alkenyl-O-alkyl, where O is not directly connected to CH₂=CH, and the hydrogen atoms on the alkenyl can be partially or completely replaced by halogen atoms, such as ClCH=CHCHCH₂OCH₂CH₂-. Ynyloxy alkyl refers to alkynyl-O-alkyl, such as CH≡CCH₂OCH₂CH₂-, where O is not directly connected to CH≡C. Halogenated ynyloxy alkyl refers to alkynyl-O-alkyl, on which the hydrogen atoms can be replaced by halogen atoms, such as ClC≡CCH₂OCH₂CH₂-. Alkyl S(O)ₙ alkyl refers to alkyl-S(O)ₙ-alkyl-, n=0, 1 or 2, such as CH₃SCH₂CH₂CH₂-, CH₃SOCH₂CH₂-, CH₃SO₂CH₂CH₂-.

The aforementioned method of the present invention can also include the necessary pretreatment of the aforementioned raw materials and the necessary post-treatment of the reaction products. The operation methods of pretreatment and post-treatment include but are not limited to drying, washing, beating, filtration, centrifugation, column chromatography, recrystallization, etc. The example part of the present invention provides several specific processing methods, which should not be understood as a limitation to the present invention.

Unless otherwise specified, the definitions of each functional group in the reaction scheme are the same as before.

The herbicide 2-chloro-4-fluoro-5-(3-methyl-2,6-dioxo-4-(trifluoromethyl)-3,6-dihydropyrimidin-1(2H)-yl) benzoate compound of formula (I) prepared by the present invention has excellent killing activity against broad-spectrum and economically important annual harmful plants of monocotyledons and dicotyledons, and can effectively control a variety of weeds. This compound can achieve good results at low doses, which can be used as herbicide.

If the name of a compound in the present invention is in conflict with the structural formula, the structural formula shall prevail unless the structural formula is obviously wrong.

The beneficial effect of the present invention is that: the synthesis idea of the present invention is to use 2-chloro-4-fluoro-5-nitrobenzoyl chloride as the starting material, first splice small segments of the side chain with the carboxyl, and finally use different methods to synthesize the uracil ring. The strategy of protecting group is avoided, the atom economy is improved, the total step of synthesis is shortened, the generation of impurities is reduced, and the utilization rate of raw materials and reagents is greatly improved. At the same time, the present invention has various ways of synthesizing the uracil ring, which provides a good idea for industrial development and is conducive to the transformation to industrial production. The raw materials and reagents are easily available, the reaction conditions are mild, the operation and post-treatment are simple, and the product yield and purity are high, which greatly reduces the cost.

### DETAILED DESCRIPTION OF THE INVENTION

Hereinafter, the present invention will be described with reference to the following but not limiting examples. Any simple replacement or improvement made to the present invention by the person skilled in the art shall fall within the protection scope of the present invention.

Several methods for preparing compounds of the present invention are explained in detail in the following schemes and examples. The raw materials can be purchased from the market or prepared using methods known in the literature or as detailed in the explanation. The person skilled in the art should understand that the compounds of the present invention can also be synthesized using other synthesis routes. Although specific raw materials and conditions in the synthesis route have been explained below, they can be easily replaced with other similar raw materials and conditions, and various isomers of the compounds prepared by variants or variations of the preparation method of the present invention shall fall within the protection scope of the present invention. In addition, the preparation method described below can be further modified according to the disclosure of the present invention, using conventional chemical methods well-known to one skilled in the art, for example, protecting appropriate groups during the reaction process, and so on.

The method examples provided below are intended to promote further understanding of the preparation method of the present invention, and the specific substances, types, and conditions used are determined as further explanations of the present invention, not as limitations to the reasonable protection scope of the present invention. The raw materials and reagents used in the synthesis of the compounds described below can either be purchased from the market or easily prepared by one skilled in the art.

The analytical instrument described in the examples is as follows:
1. High performance liquid chromatography (hereinafter referred to as HPLC):
   Method A:
      Using Agilent Technologies, 1260 Infinity II device
      Column: Agilent Eclipse Plus C18 3.5 µm. 4.6 * 100 mm
      Mobile phase: A: water+0.1% phosphoric acid; B: acetonitrile, temperature: 30 °C
      Gradient: 5% B to 95% B within 15 minutes; 95% B 3 min
      Flow rate: 1 mL/min
   Method B:
      Using Agilent Technologies, 1260 Infinity II device
      Column: Agilent Eclipse Plus C18 3.5 µ m. 4.6 * 100 mm
      Mobile phase: A: water+0.1% phosphoric acid; B: methanol, temperature: 30 °C
      Gradient: 30% B to 95% B within 20 minutes; 95% B 3 min
      Flow rate: 1 mL/min
2.Ultra high performance liquid chromatography-tandem mass spectrometry (hereinafter referred to as LC-MS): using Waters, ACQUITY H-Class UPLC-SQ Detector 2 device
   Column: ACQUITY UPLC ^{®} BEH C18 1.7 µm, 2.1 * 50 mm Column
   Mobile phase: A: water + 0.2 % formic acid; b: acetonitrile, temperature: 30 °C
   Gradient: 10 % B to 95 % B within 5 min; 95 % B 1 min
   Flow rate: 0.5 mL/min.
   MS method: ESI positive, negative, mass range (m/z): 100-800
3. Gas chromatograph (hereinafter referred to as GC): using Agilent Technologies, 7890B GC device
   Detector: FID
   Chromatographic column: HP-1 30m*530µm*10.5µm
   Injection port temperature: 250 °C
   Split ratio: 40:1
   Flow rate: 20 mL/min
   H₂: 30 mL/min
   Air: 300 mL/min
   He: 25 mL/min
   Detector temperature: 280 °C
   Method: remain the temperature of 40°C for 2min, raise the temperature to 260 °C at a rate of 20 °C/min, and remain the temperature of 260 °C for 5 min, a total time of 18 min
4. Gas chromatography-tandem mass spectrometry (hereinafter referred to as GC-MS): using Agilent Technologies, 7890B GC System-5977A MSD device
   Column: Agilent Technologies, HP-5MS UI 0.25 µm, 30m*0.250mm
   Sample injector temperature: 250 °C
   Chromatographic column flow rate: helium 1 mL/min
   Method: remain the temperature of 40 °C for 2 min, raise the temperature to 280 °C at a rate of 20 °C/min, remain the temperature of 280 °C for 5 min, a total time of 19 min
   MSD transmission line temperature: 280 °C
   EI ion source temperature: 230 °C, MS quadrupole temperature: 150°C, scanning range: 30.00-400.00

In addition, the chemical shift value of proton nuclear magnetic resonance spectrum (hereinafter referred to as ¹H-NMR) recorded below is measured at 400MHz (Bruker, AVANCE III HD 400M) in deuterated chloroform solvent using Me₄Si (tetramethylsilane) as the standard substance. In the case of determination in deuterated dimethyl sulfoxide solvent, it is shown as "(DMSO-d6)" in the data of chemical shift value. It should be noted that the symbols in the chemical shift values of ¹H-NMR represent the following meanings.

s: singlet, d: doublet, dd: double doublet, dt: double triplet, td: triple doublet, ddd: double double doublet, t: triplet, q: quartet, sep: septet, m: multiplet, brs: broad singlet. In addition, in the presence of two or more stereoisomers, each chemical shift value is marked with "and" for signals that can be analyzed.

The examples of representative compounds are as follows, and the synthesis methods of other compounds are similar, which will not be explained in detail here.

### Example 1: Synthesis of 2-methoxyethyl 2-hydroxy-2-methylpropionate

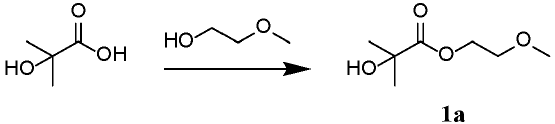

In a reaction flask, were placed 50 g (480.31 mmol) 2-hydroxyisobutyric acid, 300 g ethylene glycol monomethyl ether and 2.35 g (23.96 mmol) concentrated sulfuric acid (98%). The reaction mixture was heated to 125°C and refluxed for 4 h. After the reaction was completed, the remaining ethylene glycol monomethyl ether was removed by reduced pressure distillation, and the residue was vacuum distilled. The fraction at the distillation head temperature of 100 °C was collected to give 58.40 g product **1a** with a yield of 78% and a GC area normalized purity of 98.6 %. ¹HNMR (400 MHz, DMSO-*d*₆) δ 5.29 (s, 1H), 4.21 - 4.07 (m, 2H), 3.57 - 3.49 (m, 2H), 3.27 (s, 3H), 1.29 (s, 6H).

### Example 2: Synthesis of 2-methoxyethyl 2-hydroxy-2-methylpropionate

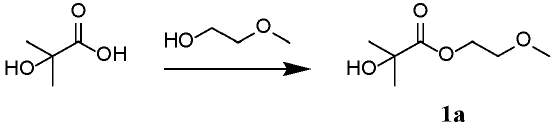

In a 500 mL four-necked flask, were placed 156.15 g (1.50 mol) 2-hydroxyisobutyric acid, 125.55 g (1.65 mol) ethylene glycol monomethyl ether, 1.43 g (7.5 mmol) p-toluenesulfonic acid monohydrate and 200 g toluene. The reaction mixture was heated and refluxed for 3-5 hours using a Dean-Stark apparatus for azeotropic removal of water until no new water was generated. The mixture was distilled at atmospheric pressure to remove toluene and the remaining ethylene glycol monomethyl ether, and then distilled at reduced pressure(-0.099 Mpa). The colorless liquid fraction at the distillation head temperature of 100°C was collected to give 231.06 g product **1a** with a yield of 94.98% and a GC area normalized purity of 98.51%.

### Example 3: Synthesis of 1-(2-methoxyethoxy)-2-methyl-1-oxopropan-2-yl 2-chloro-4-fluoro-5-nitrobenzoate

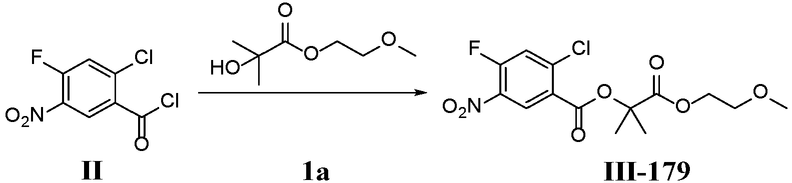

In the reaction flask, was placed 32.7 g (201.7 mmol) compound **1a**, and cooled to 0°C under ice bath. 19.9 g (252.1 mmol) pyridine was added, and 80 g dichloromethane solution of 40 g (168.1 mmol) compound **II** was added dropwise at 0-5°C under nitrogen protection. After addition, the mixture returned to room temperature to react for 1 h. The reaction was quenched by methanol to detect the content of compound **II**. The formed methyl 2-chloro-4-fluoro-5-nitrobenzoate was analyzed by HPLC (Method A). The content of methyl 2-chloro-4-fluoro-5-nitrobenzoate was less than 1% as the end of the reaction. The reaction solution was added with 80g water and stirred for 10 min, standing for layering. The organic phase was separated and the solvent was removed under reduced pressure to obtain 55.38 g light yellow oil as title compound **III-179** with a yield of 90.59% and an HPLC area normalization purity of 91.7% (Method A). ¹HNMR (400 MHz, DMSO-*d*₆) δ 8.53 (d, *J =* 8.0 Hz, 1H), 8.12 (d, *J =* 11.1 Hz, 1H), 4.27 - 4.14 (m, 2H), 3.56 - 3.48 (m, 2H), 3.22 (s, 3H), 1.66 (s, 6H).

### Example 4: Synthesis of 1-(2-methoxyethoxy)-2-methyl-1-oxopropan-2-yl 2-chloro-4-fluoro-5-nitrobenzoate

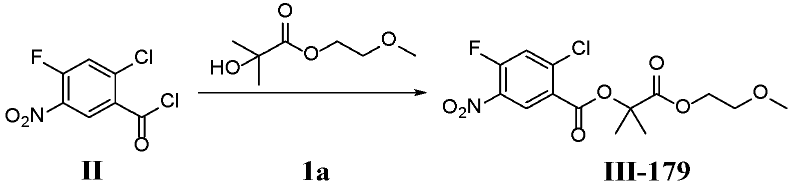

In a 2 L four-necked flask, were placed 238.0 g (1 mol) compound II and 1000 g toluene. The reaction mixture was heated 80-90°C while stirring. At this temperature, a mixed solution of 222.62 g (2.20 mol) triethylamine and 243.27 g (1.50 mol) compound **1a** was added dropwise for 2 h, and the reaction was continued for 4-5 h after addition. The reaction was quenched by methanol to detect the content of compound **II**. The formed methyl 2-chloro-4-fluoro-5-nitrobenzoate was analyzed by HPLC (Method A). The content of methyl 2-chloro-4-fluoro-5-nitrobenzoate was less than 1% as the end of the reaction. The reaction solution was cooled to room temperature and added with 700 g ice water while stirring, standing for layering. The organic phase was distilled under reduced pressure to remove the solvent, and 361.21 g brown oil was obtained, which was compound **III-179**. The crude yield was 99.31%, and HPLC area normalization purity was 92.51% (Method A).

### Example 5: Synthesis of 1-(2-methoxyethoxy)-2-methyl-1-oxopropan-2-yl 2-chloro-4-fluoro-5-nitrobenzoate

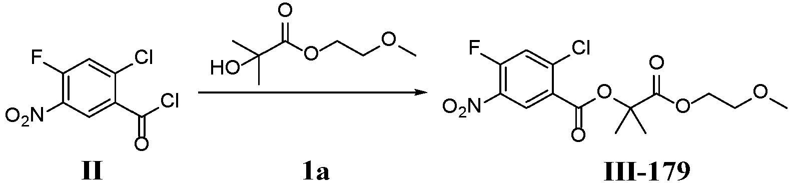

In a 1 L four-necked flask, were placed 101.19 g (1 mol) triethylamine, 3.05 g (0.025 mol) 4-dimethylaminopyridine, 121.64 g (0.75 mol) compound **1a** and 200 g toluene. The mixture was heated to 80°C while stirring. The mixture of 119.0 g (0.5 mol) compound **II** and 300 g toluene was added dropwise at 80°C for 2 h, and the reaction was continued at 80°C for 4 h after addition. The reaction was quenched by methanol to detect the content of compound **II**. The formed methyl 2-chloro-4-fluoro-5-nitrobenzoate was analyzed by HPLC (Method A). The content of methyl 2-chloro-4-fluoro-5-nitrobenzoate was less than 1% as the end of the reaction. The reaction solution was cooled to room temperature and added with 500 g ice water while stirring, standing for layering. The organic phase was distilled under reduced pressure to remove the solvent to give 173.06 g brown oil, which was compound **III-179**. The yield of crude product was 95.16%, and HPLC area normalization purity was 87.90% (Method A).

### Example 6: Synthesis of 1-(2-methoxyethoxy)-2-methyl-1-oxopropan-2-yl 2-chloro-4-fluoro-5-nitrobenzoate

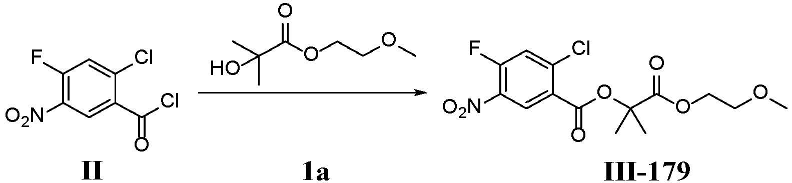

In a 1 L four-necked flask, were placed 119.0 g (0.5 mol) compound **II** and 300 g toluene. The mixture was heated to 80°C while stirring, a mixture of 101.19 g (1 mol) triethylamine, 3.05 g (0.025 mol) 4-dimethylaminopyridine, 121.64 g (0.75 mol) compound **1a** and 200 g toluene was added dropwise at 80°C for 2 h, and then the reaction was continued at 80°C for 4 h after addition. The reaction was quenched by methanol to detect the content of compound **II**. The formed methyl 2-chloro-4-fluoro-5-nitrobenzoate was analyzed by HPLC (Method A). The content of methyl 2-chloro-4-fluoro-5-nitrobenzoate was less than 1% as the end of the reaction. The reaction solution was cooled to room temperature and added with 500 g ice water while stirring, standing for layering. The organic phase was distilled under reduced pressure to remove the solvent to give 175.20 g brown oil, which was compound **III-179**. The yield of crude product was 96.34%, and HPLC area normalization purity was 92.00% (Method A).

### Example 7: Synthesis of 1-(2-methoxyethoxy)-2-methyl-1-oxopropan-2-yl 5-amino-2-chloro-4-fluorobenzoate

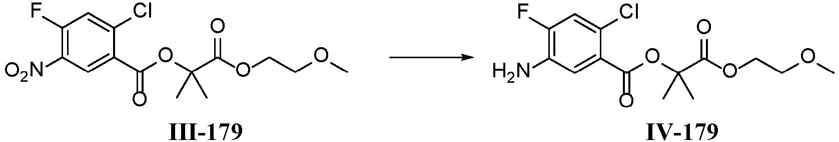

100 g compound **III-179** was dissolved in 500 g methanol, which was added to a 1000 mL autoclave, then 3 g Pt/C (1%) was added. The gas in the autoclave was replaced 5 times with hydrogen. The reaction solution was heated to 45°C and the hydrogen pressure in the autoclave was controlled at 2.0 MPa to react for 8 h. The raw material compound **III-179** was detected by sampling until it disappeared, and then the insoluble matter was removed by filtration. The filtrate was distilled under reduced pressure to remove the solvent to give 87.72 g brown oil, which was title compound **IV-179** with a yield of 95.6% and HPLC area normalization purity of 96.2% (Method A). ¹H NMR (400 MHz, DMSO-*d*₆) δ 7.30 (d, *J =* 11.1 Hz, 1H), 7.24 (d, *J =* 9.4 Hz, 1H), 4.23 - 4.18 (m, 2H), 3.54 - 3.49 (m, 2H), 3.22 (s, 3H), 1.60 (s, 6H).

### Example 8: Synthesis of 1-(2-methoxyethoxy)-2-methyl-1-oxopropan-2-yl 5-amino-2-chloro-4-fluorobenzoate

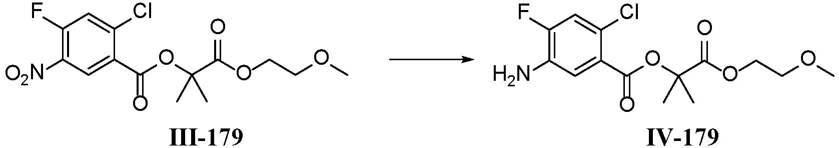

In a 2 L autoclave, were place 361.21 g (0.93 mol) compound **III-179** obtained in example 4 which was dissolved in 1000 g methanol and 3.6 g Pt/C (5%). The hydrogen pressure was 2.0 MPa. The reaction solution was heated to 60-70°C for 5-6 h, and the content of compound **III-179** detected by sampling and HPLC (Method A) was less than 0.5% as the end of the reaction. Pt/C was removed by filtration, and the filtrate was distilled under reduced pressure to obtain crude product. The crude product was added to 200 g 70% (w/w) isopropanol aqueous solution, heated to 60°C and stirred for 0.5 h, slowly cooled to about 0°C and remained the temperature of 0 °C for more than 2 h to precipitate crystals. The mixture was filtered, the filter cake was collected and dried to obtain 267.31 g yellow solid, which was the title compound **IV-179**. The two-step yield (calculated as compound II in example 4) was 80.10%, and HPLC area normalization purity (Method A) was 98.51%.

### Example 9: Synthesis of 1-(2-methoxyethoxy)-2-methyl-1-oxopropan-2-yl 2-chloro-5-(2,6-dioxo-4-(trifluoromethyl)-3,6-dihydropyrimidin-1(2H)-yl)-4-fluorobenzoate

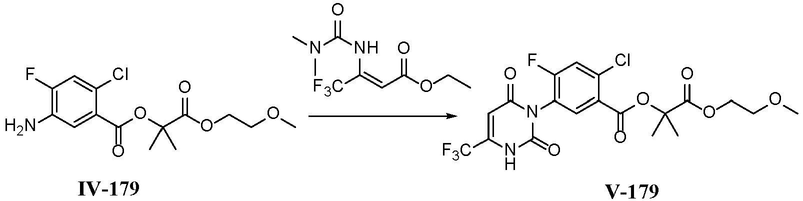

In a reaction flask, were placed 50 g (149.82 mmol) compound **IV-179**, 41.89 g (164.8 mmol) ethyl 3-(3,3-dimethylureido)-4,4,4-trifluorobut-2-enoate, and 250 g acetic acid. The mixture were heated to 110°C to react for 4 h, and the raw material **IV-179** disappeared by detection. After removing the remaining acetic acid by reduced pressure distillation, water and ethyl acetate were added for extraction. The organic phase was washed twice with water, and was distilled under reduced pressure to remove the solvent to give 71.6 g the title compound **V-179**, with a yield of 96.2% and HPLC area normalization purity of 92.7% (Method B). It is a light yellow solid with a melting point of 142.4°C-143.3°C.¹H NMR (400 MHz, DMSO-d*6*) δ 12.85 (s, 1H), 8.09 (d, *J =* 7.7 Hz, 1H), 7.90 (d, *J =* 9.5 Hz, 1H), 6.43 (s, 1H), 4.24 - 4.19 (m, 2H), 3.53 - 3.49 (m, 2H), 3.20 (s, 3H), 1.63 (s, 6H).

### Example 10: Synthesis of 1-(2-methoxyethoxy)-2-methyl-1-oxopropan-2-yl 2-chloro-5-(2,6-dioxo-4-(trifluoromethyl)-3,6-dihydropyrimidin-1(2H)-yl)-4-fluorobenzoate

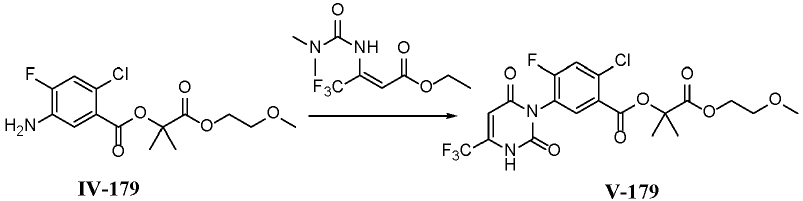

In a 100 mL four-necked flask, were placed 10.00 g (0.03 mol) compound **IV-179**, 30 g acetic acid, and 9.2 g (0.05 mol) ethyl 3-(3,3-dimethylureido)-4,4,4-trifluorobut-2-enoate. The mixture was heated to reflux for 4h, and compound **IV-179** disappeared or was less than 1% by HPLC detection (Method B). The reaction mixture was distilled under reduced pressure to remove acetic acid to give 14.09 g brown oil which was compound **V-179**, with a yield of 94.54% and HPLC area normalization purity of 92.51% (Method B).

### Example 11: Synthesis of 1-(2-methoxyethoxy)-2-methyl-1-oxopropan-2-yl 2-chloro-5-(2,6-dioxo-4-(trifluoromethyl)-3,6-dihydropyrimidin-1(2H)-yl)-4-fluorobenzoate

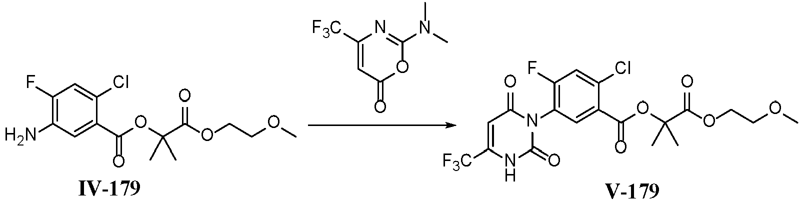

Refer to the synthesis method of compound **V-179** in example 9 and example 10. In a 250 mL four-necked flask, were placed 16.70 g (0.05 mol) compound **IV-179**, 100 g acetic acid and 12.50 g (0.06 mol) 2-(dimethylamino)-4-(trifluoromethyl)-6H-1,3-oxazin-6-one. The mixture was heated to reflux for 4 h, and compound **IV-179** disappeared or was less than 1% by HPLC detection (Method B). The reaction mixture was distilled under reduced pressure to remove acetic acid to give 22.75 g brown oil which was compound **V-179**, with a yield of 91.59% and HPLC area normalization purity of 75.51% (Method B).

Through the method of the present invention, the prepared compound of formula **(V)** can be subjected to the following reaction to prepare the herbicidal compound of formula (I):

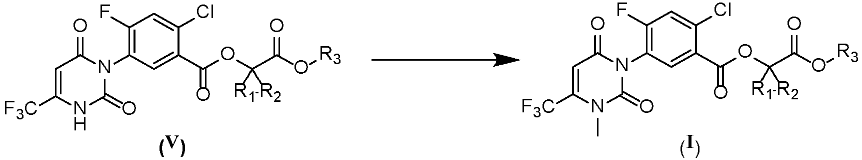

### Example 12: Synthesis of 1-(2-methoxyethoxy)-2-methyl-1-oxopropan-2-yl 2-chloro-4-fluoro-5-(3-methyl-2,6-dioxo-4-(trifluoromethyl)-3,6-dihydropyrimidin-1(2H)-yl) benzoate

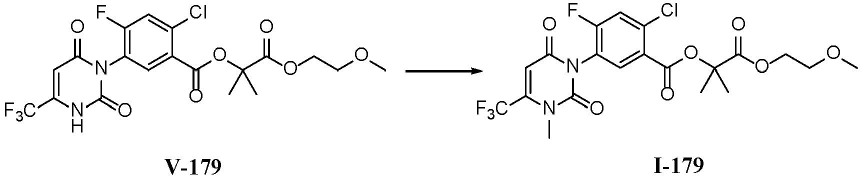

In a reaction flask, were placed 100 g (201.28 mmol) compound V-179, 58.42 g (422.7 mmol) potassium carbonate and 500 g N,N-dimethylformamide. The mixture was stirred at room temperature for 0.5 h, and 27.93 g (221.42mmol) dimethyl sulfate was added dropwise slowly. After addition, the reaction continued until the material compound **V-179** disappeared by detection. The reaction solution was poured into 500 g water, extracted with 1000 g ethyl acetate. The organic phase was washed twice with saturated brine and spin-dried to give 94.76 g title compound **1-179**, with a yield of 92% and HPLC area normalization purity of 93.7% (Method B). ¹H NMR (400 MHz, DMSO-*d*₆) δ 8.07 (d, *J =* 7.7 Hz, 1H), 7.92 (d, *J =* 9.5 Hz, 1H), 6.63 (s, 1H), 4.24 - 4.19 (m, 2H), 3.54 - 3.48 (m, 2H), 3.43 (s, 3H), 3.21 (s, 3H), 1.63 (s, 6H).

### Example 13: Synthesis of 1-(2-methoxyethoxy)-2-methyl-1-oxopropan-2-yl 2-chloro-4-fluoro-5-(3-methyl-2,6-dioxo-4-(trifluoromethyl)-3,6-dihydropyrimidin-1(2H)-yl) benzoate

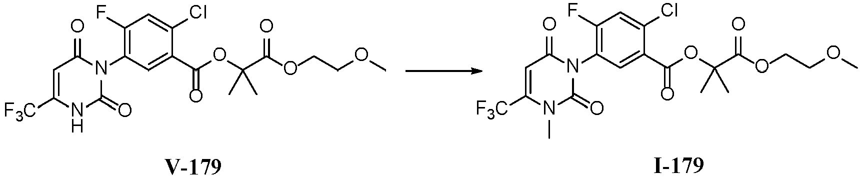

In a 250 mL four-necked flask, were placed 10 g (20 mmol) compound **V-179**, 50 g N,N-dimethylformamide and 3.5 g (25 mmol) potassium carbonate, and 3.5 g (25 mmol) iodomethane was added dropwise at room temperature. After addition, the mixture was stirred at room temperature for 3 h, and compound **V-179** disappeared or was less than 1% by HPLC detection (Method B). The reaction mixture was distilled under reduced pressure to remove most of the solvent, and was added with 100 g water and 100 g ethyl acetate for extraction. The organic phase was distilled under reduced pressure to remove the solvent to give a light yellow semisolid, which was recrystallized with isopropanol to give 9.2 g white solid of compound **I-179**, with a yield of 90% and HPLC area normalized purity of 98.67% (Method B).

### Example 14: Synthesis of 1-(2-methoxyethoxy)-2-methyl-1-oxopropan-2-yl 2-chloro-4-fluoro-5-(3-methyl-2,6-dioxo-4-(trifluoromethyl)-3,6-dihydropyrimidin-1(2H)-yl) benzoate

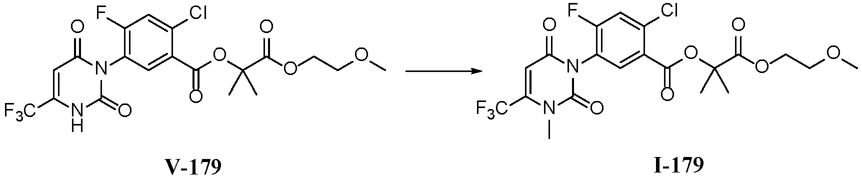

In a 250 mL four-necked flask, were placed 10 g (20 mmol) compound V-179, 50 g N,N-dimethylformamide and 3.5 g (25 mmol) potassium carbonate, and 4.6 g (48 mmol) bromomethane was added dropwise under ice bath condition. After addition, the mixture was heated to room temperature and stirred for 7 h, and compound **V-179** disappeared or was less than 1% by HPLC detection (Method B). The reaction mixture was distilled under reduced pressure to remove most of the solvent, and was added with 100 g water and 100 g ethyl acetate for extraction. The organic phase was distilled under reduced pressure to remove the solvent to give a light yellow semisolid, which was recrystallized with isopropanol to give 9.0 g white solid of compound **I-179,** with a yield of 88% and HPLC area normalized purity of 98.54% (Method B).

According to the method described above, 2-hydroxyisobutyric acid and ethylene glycol monomethyl ether in example 1 or example 2 can be replaced with corresponding R₁, R₂ substituted glycolic acid and R₃-OH to obtain other compounds of formula (I). For example, compound I-169 can be prepared by DL-lactic acid and 2-(methylthio) ethanol, which needs no further elaboration. The corresponding R₁, R₂ substituted glycolic acid and R₃-OH can be obtained by commercial purchase or can be easily prepared by one skilled in the art.

Some of the compounds of formula(I) prepared are shown in Table 4

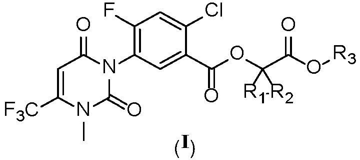

**Table 4 Structure of some compounds with general formula (I)**

| NO. | R₁ | R₂ | R₃ |
|---|---|---|---|
| **I-1** | H | H | 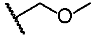 |
| **I-2** | H | H | 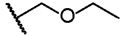 |
| **I-3** | H | H | 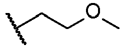 |
| **I-4** | H | H | 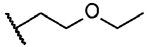 |
| **I-5** | H | H | 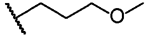 |
| **I-6** | H | H | 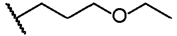 |
| **I-7** | H | H | 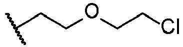 |
| **I-8** | H | H | 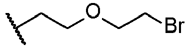 |
| **I-9** | H | H | 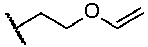 |
| **I-10** | H | H | 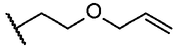 |
| **I-11** | H | H | 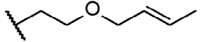 |
| **I-12** | H | H | 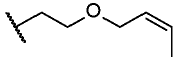 |
| **I-13** | H | H | 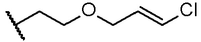 |
| **I-14** | H | H | 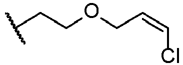 |
| **I-15** | H | H | 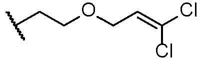 |
| **I-16** | H | H | 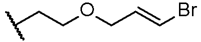 |
| **I-17** | H | H | 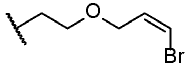 |
| **I-18** | H | H | 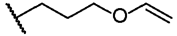 |
| **I-19** | H | H | 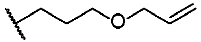 |
| **I-20** | H | H | 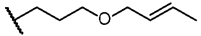 |
| **I-21** | H | H | 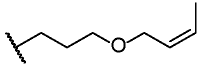 |
| **I-22** | H | H | 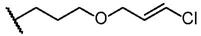 |
| **I-23** | H | H | 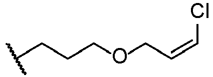 |
| **I-24** | H | H | 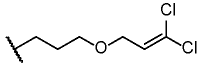 |
| **I-25** | H | H | 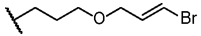 |
| **I-26** | H | H | 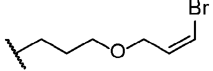 |
| **I-27** | H | H | 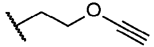 |
| **I-28** | H | H | 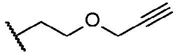 |
| **I-29** | H | H | 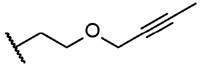 |
| **I-30** | H | H | 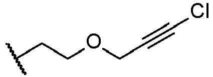 |
| **I-31** | H | H | 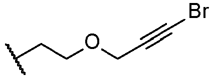 |
| **I-32** | H | H | 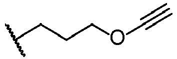 |
| **I-33** | H | H | 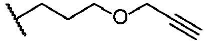 |
| **I-34** | H | H | 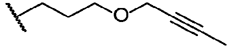 |
| **I-35** | H | H | 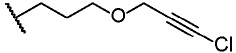 |
| **I-36** | H | H | 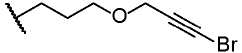 |
| **I-37** | H | H | 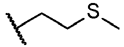 |
| **I-38** | H | H | 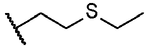 |
| **I-39** | H | H | 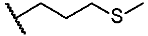 |
| **I-40** | H | H | 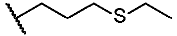 |
| **I-41** | H | H | 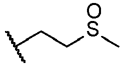 |
| **I-42** | H | H | 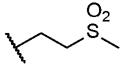 |
| **I-43** | H | H | 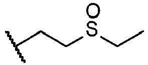 |
| **I-44** | H | H | 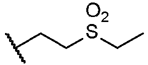 |
| **I-45** | H | 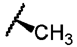 | 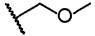 |
| **I-46** | H | 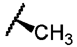 | 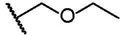 |
| **I-47** | H | 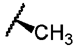 | 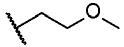 |
| **I-48** | H | 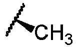 | 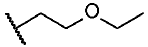 |
| **I-49** | H | 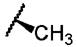 | 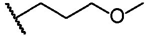 |
| **I-50** | H | 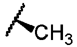 | 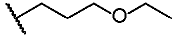 |
| **I-51** | H | 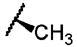 | 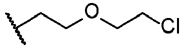 |
| **I-52** | H | 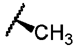 | 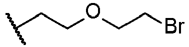 |
| **I-53** | H | 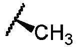 | 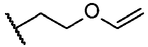 |
| **I-54** | H | 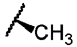 | 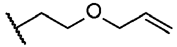 |
| **I-55** | H | 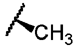 | 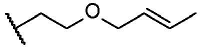 |
| **I-56** | H | 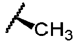 | 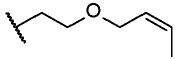 |
| **I-57** | H | 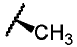 | 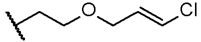 |
| **I-58** | H | 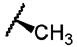 | 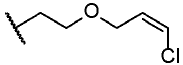 |
| **I-59** | H | 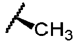 | 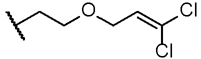 |
| **I-60** | H | 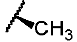 | 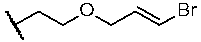 |
| **I-61** | H | 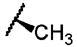 | 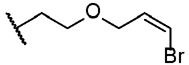 |
| **I-62** | H | 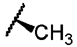 | 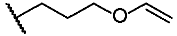 |
| **I-63** | H | 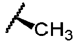 | 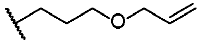 |
| **I-64** | H | 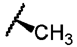 | 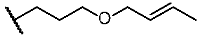 |
| **I-65** | H | 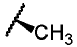 | 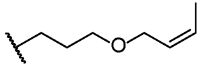 |
| **I-66** | H | 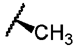 | 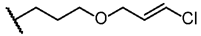 |
| **I-67** | H | 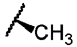 | 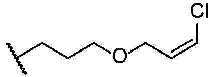 |
| **I-68** | H | 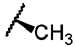 | 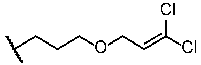 |
| **I-69** | H | 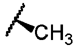 | 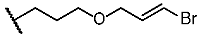 |
| **I-70** | H | 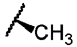 | 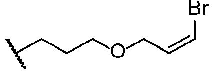 |
| **I-71** | H | 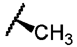 | 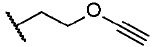 |
| **I-72** | H | 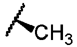 | 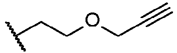 |
| **I-73** | H | 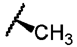 | 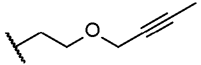 |
| **I-74** | H | 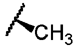 | 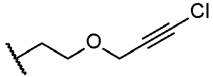 |
| **I-75** | H | 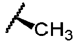 | 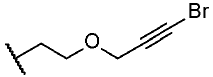 |
| **I-76** | H | 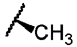 | 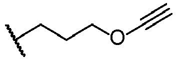 |
| **I-77** | H | 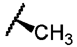 | 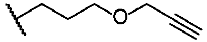 |
| **I-78** | H | 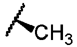 | 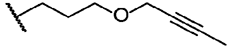 |
| **I-79** | H | 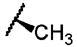 | 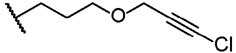 |
| **I-80** | H | 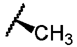 | 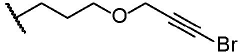 |
| **I-81** | H | 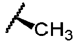 | 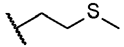 |
| **I-82** | H | 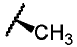 | 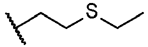 |
| **I-83** | H | 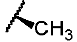 | 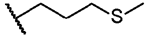 |
| **I-84** | H | 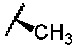 | 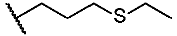 |
| **I-85** | H | 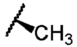 | 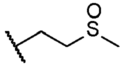 |
| **I-86** | H | 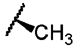 | 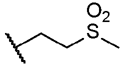 |
| **I-87** | H | 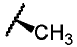 | 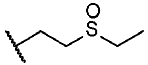 |
| **I-88** | H | 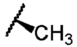 | 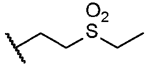 |
| **I-89** | H | 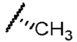 | 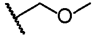 |
| **I-90** | H | 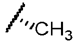 | 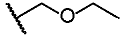 |
| **I-91** | H | 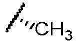 | 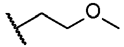 |
| **I-92** | H | 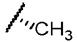 | 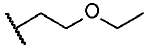 |
| **I-93** | H | 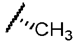 | 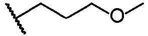 |
| **I-94** | H | 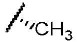 | 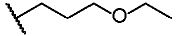 |
| **I-95** | H | 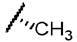 | 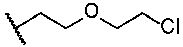 |
| **I-96** | H | 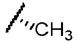 | 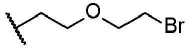 |
| **I-97** | H | 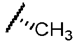 | 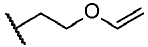 |
| **I-98** | H | 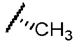 | 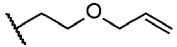 |
| **I-99** | H | 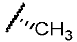 | 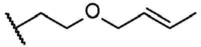 |
| **I-100** | H | 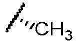 | 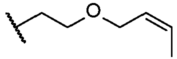 |
| **I-101** | H | 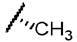 | 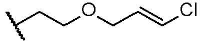 |
| **I-102** | H | 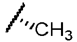 | 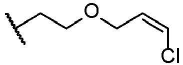 |
| **I-103** | H | 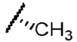 | 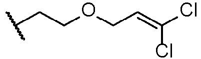 |
| **I-104** | H | 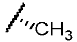 | 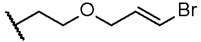 |
| **I-105** | H | 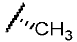 | 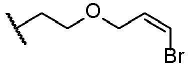 |
| **I-106** | H | 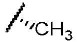 | 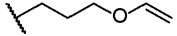 |
| **I-107** | H | 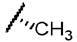 | 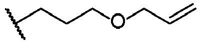 |
| **I-108** | H | 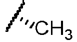 | 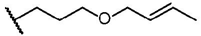 |
| **I-109** | H | 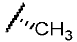 | 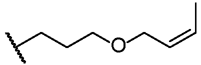 |
| **I-110** | H | 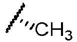 | 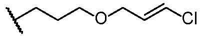 |
| **I-111** | H | 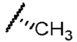 | 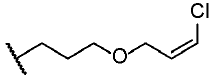 |
| **I-112** | H | 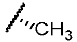 | 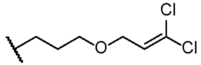 |
| **I-113** | H | 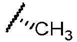 | 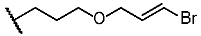 |
| **I-114** | H | 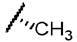 | 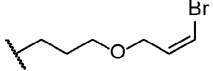 |
| **I-115** | H | 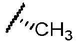 | 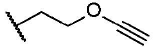 |
| **I-116** | H | 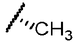 | 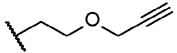 |
| **I-117** | H | 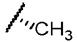 | 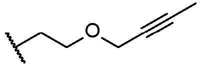 |
| **I-118** | H | 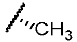 | 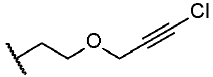 |
| **I-119** | H | 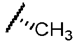 | 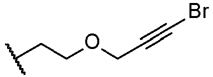 |
| **I-120** | H | 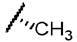 | 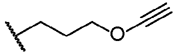 |
| **I-121** | H | 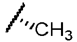 | 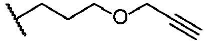 |
| **I-122** | H | 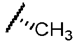 | 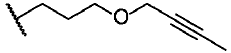 |
| **I-123** | H | 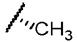 | 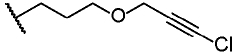 |
| **I-124** | H | 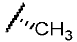 | 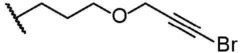 |
| **I-125** | H | 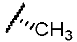 | 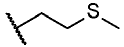 |
| **I-126** | H | 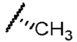 | 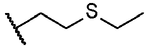 |
| **I-127** | H | 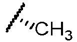 | 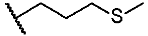 |
| **I-128** | H | 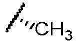 | 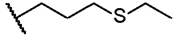 |
| **I-129** | H | 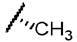 | 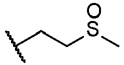 |
| **I-130** | H | 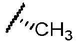 | 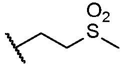 |
| **I-131** | H | 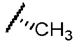 | 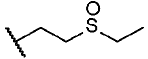 |
| **I-132** | H | 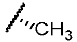 | 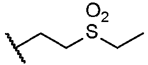 |
| **I-133** | H | CH₃ | 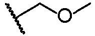 |
| **I-134** | H | CH₃ | 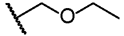 |
| **I-135** | H | CH₃ | 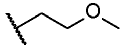 |
| **I-136** | H | CH₃ | 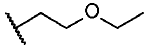 |
| **I-137** | H | CH₃ | 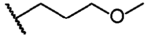 |
| **I-138** | H | CH₃ | 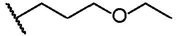 |
| **I-139** | H | CH₃ | 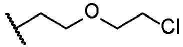 |
| **I-140** | H | CH₃ | 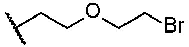 |
| **I-141** | H | CH₃ | 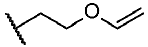 |
| **I-142** | H | CH₃ | 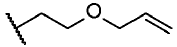 |
| **I-143** | H | CH₃ | 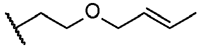 |
| **I-144** | H | CH₃ | 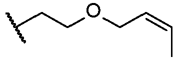 |
| **I-145** | H | CH₃ | 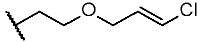 |
| **I-146** | H | CH₃ | 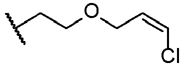 |
| **I-147** | H | CH₃ | 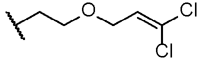 |
| **I-148** | H | CH₃ | 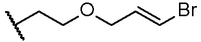 |
| **I-149** | H | CH₃ | 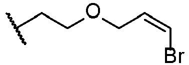 |
| **I-150** | H | CH₃ | 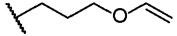 |
| **I-151** | H | CH₃ | 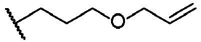 |
| **I-152** | H | CH₃ | 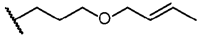 |
| **I-153** | H | CH₃ | 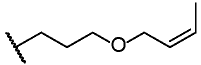 |
| **I-154** | H | CH₃ | 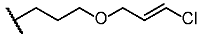 |
| **I-155** | H | CH₃ | 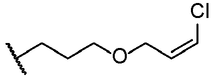 |
| **I-156** | H | CH₃ | 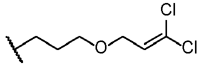 |
| **I-157** | H | CH₃ | 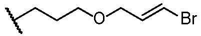 |
| **I-158** | H | CH₃ | 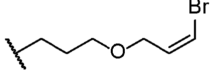 |
| **I-159** | H | CH₃ | 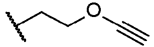 |
| **I-160** | H | CH₃ | 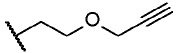 |
| **I-161** | H | CH₃ | 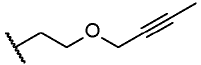 |
| **I-162** | H | CH₃ | 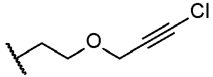 |
| **I-163** | H | CH₃ | 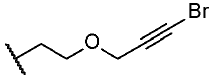 |
| **I-164** | H | CH₃ | 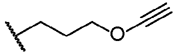 |
| **I-165** | H | CH₃ | 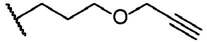 |
| **I-166** | H | CH₃ | 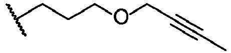 |
| **I-167** | H | CH₃ | 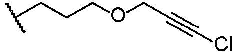 |
| **I-168** | H | CH₃ | 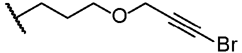 |
| **I-169** | H | CH₃ | 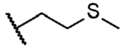 |
| **I-170** | H | CH₃ | 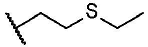 |
| **I-171** | H | CH₃ | 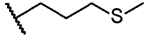 |
| **I-172** | H | CH₃ | 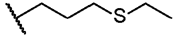 |
| **I-173** | H | CH₃ | 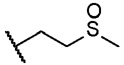 |
| **I-174** | H | CH₃ | 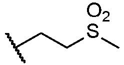 |
| **I-175** | H | CH₃ | 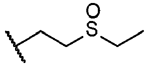 |
| **I-176** | H | CH₃ | 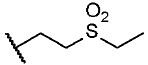 |
| **I-177** | CH₃ | CH₃ | 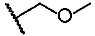 |
| **I-178** | CH₃ | CH₃ | 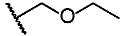 |
| **I-179** | CH₃ | CH₃ | 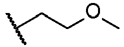 |
| **I-180** | CH₃ | CH₃ | 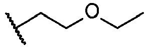 |
| **I-181** | CH₃ | CH₃ | 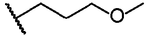 |
| **I-182** | CH₃ | CH₃ | 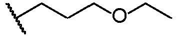 |
| **I-183** | CH₃ | CH₃ | 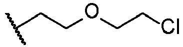 |
| **I-184** | CH₃ | CH₃ | 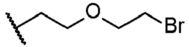 |
| **I-185** | CH₃ | CH₃ | 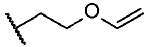 |
| **I-186** | CH₃ | CH₃ | 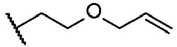 |
| **I-187** | CH₃ | CH₃ | 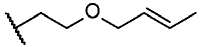 |
| **I-188** | CH₃ | CH₃ | 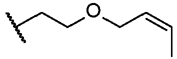 |
| **I-189** | CH₃ | CH₃ | 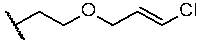 |
| **I-190** | CH₃ | CH₃ | 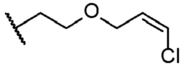 |
| **I-191** | CH₃ | CH₃ | 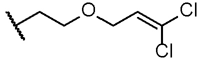 |
| **I-192** | CH₃ | CH₃ | 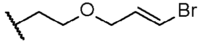 |
| **I-193** | CH₃ | CH₃ | 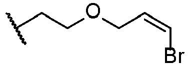 |
| **I-194** | CH₃ | CH₃ | 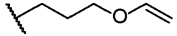 |
| **I-195** | CH₃ | CH₃ | 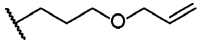 |
| **I-196** | CH₃ | CH₃ | 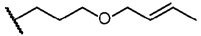 |
| **I-197** | CH₃ | CH₃ | 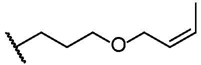 |
| **I-198** | CH₃ | CH₃ | 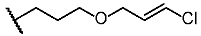 |
| **I-199** | CH₃ | CH₃ | 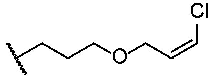 |
| **I-200** | CH₃ | CH₃ | 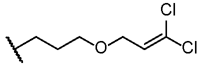 |
| **I-201** | CH₃ | CH₃ | 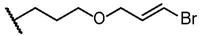 |
| **I-202** | CH₃ | CH₃ | 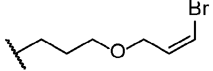 |
| **I-203** | CH₃ | CH₃ | 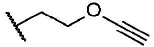 |
| **I-204** | CH₃ | CH₃ | 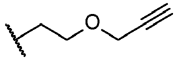 |
| **I-205** | CH₃ | CH₃ | 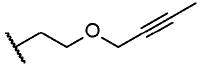 |
| **I-206** | CH₃ | CH₃ | 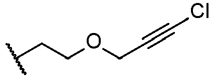 |
| **I-207** | CH₃ | CH₃ | 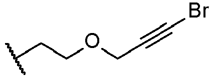 |
| **I-208** | CH₃ | CH₃ | 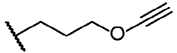 |
| **I-209** | CH₃ | CH₃ | 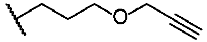 |
| **I-210** | CH₃ | CH₃ | 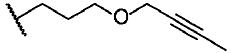 |
| **I-211** | CH₃ | CH₃ | 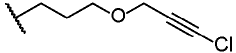 |
| **I-212** | CH₃ | CH₃ | 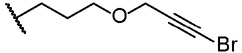 |
| **I-213** | CH₃ | CH₃ | 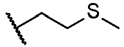 |
| **I-214** | CH₃ | CH₃ | 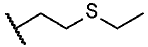 |
| **I-215** | CH₃ | CH₃ | 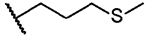 |
| **I-216** | CH₃ | CH₃ | 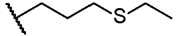 |
| **I-217** | CH₃ | CH₃ | 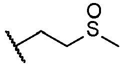 |
| **I-218** | CH₃ | CH₃ | 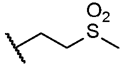 |
| **I-219** | CH₃ | CH₃ | 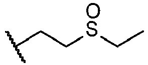 |
| **I-220** | CH₃ | CH₃ | 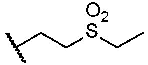 |
| **I-221** | -CH₂CH₂- | | 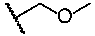 |
| **I-222** | -CH₂CH₂- | | 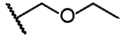 |
| **I-223** | -CH₂CH₂- | | 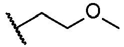 |
| **I-224** | -CH₂CH₂- | | 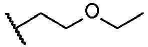 |
| **I-225** | -CH₂CH₂- | | 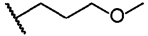 |
| **I-226** | -CH₂CH₂- | | 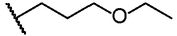 |
| **I-227** | -CH₂CH₂- | | 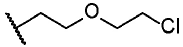 |
| **I-228** | -CH₂CH₂- | | 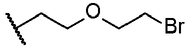 |
| **I-229** | -CH₂CH₂- | | 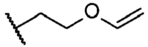 |
| **I-230** | -CH₂CH₂- | | 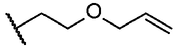 |
| **I-231** | -CH₂CH₂- | | 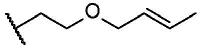 |
| **I-232** | -CH₂CH₂- | | 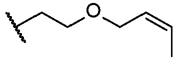 |
| **I-233** | -CH₂CH₂- | | 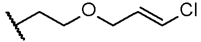 |
| **I-234** | -CH₂CH₂- | | 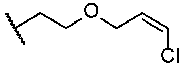 |
| **I-235** | -CH₂CH₂- | | 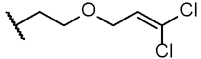 |
| **I-236** | -CH₂CH₂- | | 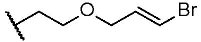 |
| **I-237** | -CH₂CH₂- | | 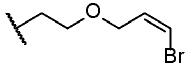 |
| **I-238** | -CH₂CH₂- | | 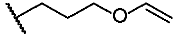 |
| **I-239** | -CH₂CH₂- | | 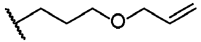 |
| **I-240** | -CH₂CH₂- | | 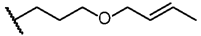 |
| **I-241** | -CH₂CH₂- | | 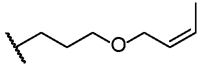 |
| **I-242** | -CH₂CH₂- | | 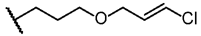 |
| **I-243** | -CH₂CH₂- | | 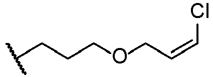 |
| **I-244** | -CH₂CH₂- | | 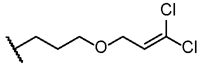 |
| **I-245** | -CH₂CH₂- | | 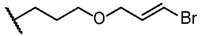 |
| **I-246** | -CH₂CH₂- | | 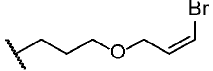 |
| **I-247** | -CH₂CH₂- | | 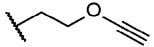 |
| **I-248** | -CH₂CH₂- | | 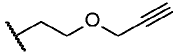 |
| **I-249** | -CH₂CH₂- | | 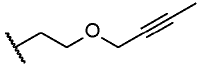 |
| **I-250** | -CH₂CH₂- | | 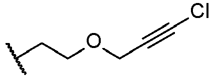 |
| **I-251** | -CH₂CH₂- | | 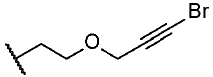 |
| **I-252** | -CH₂CH₂- | | 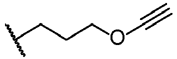 |
| **I-253** | -CH₂CH₂- | | 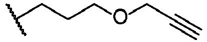 |
| **I-254** | -CH₂CH₂- | | 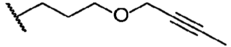 |
| **I-255** | -CH₂CH₂- | | 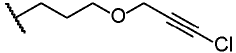 |
| **I-256** | -CH₂CH₂- | | 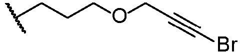 |
| **I-257** | -CH₂CH₂- | | 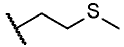 |
| **I-258** | -CH₂CH₂- | | 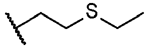 |
| **I-259** | -CH₂CH₂- | | 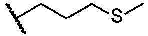 |
| **I-260** | -CH₂CH₂- | | 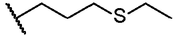 |
| **I-261** | -CH₂CH₂- | | 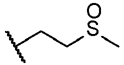 |
| **I-262** | -CH₂CH₂- | | 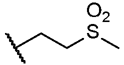 |
| **I-263** | -CH₂CH₂- | | 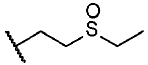 |
| **I-264** | -CH₂CH₂- | | 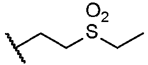 |

The above-mentioned examples are only preferred examples of the present invention. It should be pointed out that for one skilled in the art, some changes and improvements can be made without deviating from the idea of the present invention, which should be within the protection scope of the present invention.

## Claims

1. A method for preparing a uracil compound containing a carboxylate fragment with the following reaction scheme, comprising: 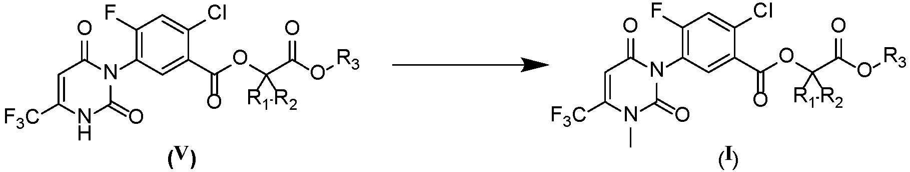
reacting the compound of formula (V) with a methylation reagent in the presence of a base in an organic solvent at -20 °C to the boiling point of the solvent to obtain a uracil compound of formula (I);
wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99: 1.

2. The method as recited in claim 1, wherein the base is selected from 4-dimethylaminopyridine, triethylamine, diisopropylethylamine, pyridine, 2-methylpyridine, 1,8-diazabicyclo[5.4.0]undec-7-ene(DBU), 2,6-Lutidine, NaH, NaNH₂, NaHCO₃, Na₂CO₃, K₂CO₃, KHCO₃, Cs₂CO₃, NaOH, LiOH, or KOH;
the methylation reagent is selected from dimethyl sulfate, chloromethane, methyl bromide, methyl iodide, methyl p-toluenesulfonate ,or methyl trifluoromethanesulfonate.

3. A method for preparing a uracil compound containing a carboxylate fragment of formula (V) with the following reaction scheme, comprising: 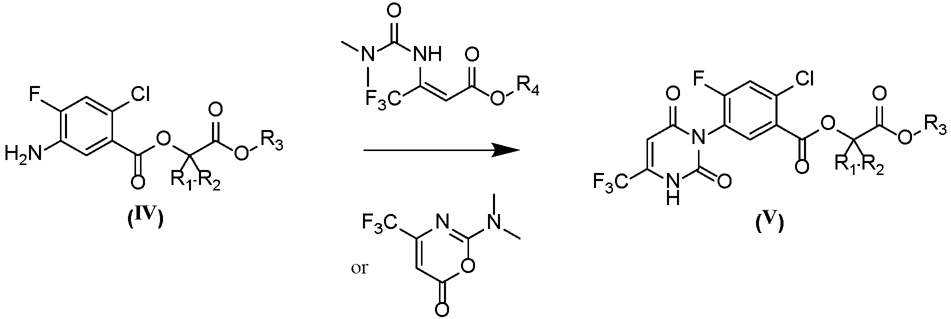
reacting the compound of formula (IV) with R₄ 3-(3,3-dimethylureido)-4,4,4-trifluorobut-2-enoate or 2-(dimethylamino)-4-(trifluoromethyl)-6H-1,3-oxazin-6-one in an organic solvent at -20 °C to the boiling point of the solvent in the presence of an acid to obtain a compound of formula (V);
wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99: 1;
R₄ is selected from methyl or ethyl.

4. The method as recited in claim 3, wherein the acid is selected from formic acid, acetic acid, trifluoroacetic acid, p-toluenesulfonic acid, sulfuric acid, hydrochloric acid, phosphoric acid, or nitric acid.

5. A method for preparing an aniline compound containing a carboxylate fragment of formula (IV) with the following reaction scheme, comprising: 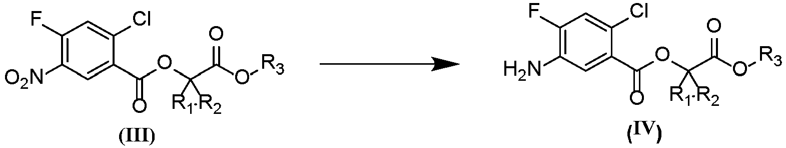
reacting the compound of formula (III) with a reductant in water or an organic solvent at -20 °C to the boiling point of the solvent to obtain a compound of formula (IV);
wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

6. The method as recited in claim 5, wherein the reductant is selected from hydrogen, metal hydride, semimetal hydride and its derivatives, reduced iron powder or reduced zinc powder.

7. A method for preparing a nitrobenzene compound containing a carboxylate fragment of formula (III) with the following reaction scheme, comprising: 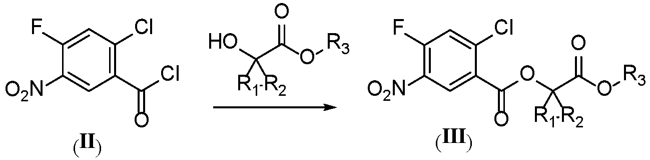
reacting the compound of formula (II) with a substituted hydroxyacetate in the presence of a base in an organic solvent at -20 °C to the boiling point of the solvent to obtain a compound of formula (III);
wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

8. The method as recited in claim 7, wherein the base is selected from 4-dimethylaminopyridine, triethylamine, diisopropylethylamine, pyridine, 2-methylpyridine, 1,8-diazabicyclo[5.4.0]undec-7-ene(DBU), 2,6-Lutidine, NaH, NaNH₂, NaHCO₃, Na₂CO₃, K₂CO₃, KHCO₃, Cs₂CO₃, NaOH, LiOH, or KOH.

9. The method as recited in any one of claims 1, 3, 5 and 7, wherein the organic solvent is one or more of alcohol, alkane, chloroalkane, ether, ester, aromatic hydrocarbon, halogenated aromatic hydrocarbon, ketone, acetonitrile, N,N-dimethylformamide, N,N-dimethylacetamide, or dimethyl sulfoxide.

10. The method as recited in claim 9, wherein the organic solvent is one or more of methanol, ethanol, isopropanol, butanol, tert-butanol, cyclohexanol, ethylene glycol, pentane, n-hexane, cyclohexane, heptane, octane, dichloromethane, 1,2-dichloroethane, chloroform, carbon tetrachloride, petroleum ether, ether, methyl tert-butyl ether, ethylene glycol dimethyl ether, tetrahydrofuran, 1,4-dioxane, ethyl acetate, butyl acetate, benzene, toluene, o-xylene, m-xylene, p-xylene, xylene, chlorobenzene, acetone, butanone, 4-methyl-2-pentanone, cyclohexanone, N-methylpyrrolidone, acetonitrile, N,N-dimethylformamide, N, N-dimethylacetamide, or dimethyl sulfoxide.

11. A uracil compound containing a carboxylate fragment of formula (V): 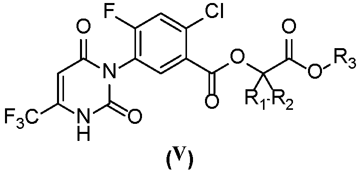
wherein R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

12. An aniline compound containing a carboxylate fragment of formula (IV): 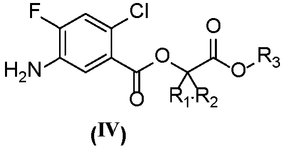 wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

13. A nitrobenzene compound containing a carboxylate fragment of formula (III): 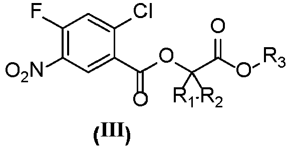 wherein,
R₁ and R₂ are each independently selected from hydrogen or methyl; or R₁ and R₂ together with the carbon atom to which they are attached form a three-membered carbocycle;
R₃ is selected from C_{1~3} alkoxy-C_{1~3} alkyl, C_{1~3} halogenated alkoxy-C_{1~3} alkyl, C_{2~6} enyloxy-C_{1~3} alkyl, C_{2~6} halogenated enyloxy-C_{1~3} alkyl, C_{2~6} ynyloxy-C_{1~3} alkyl, or C_{2~6} halogenated ynyloxy-C_{1~3} alkyl;
when R₁ is selected from hydrogen and R₂ is selected from methyl, the chiral carbon atom connected thereto is selected from either an R configuration or an S configuration, or a mixture of the two; and in the mixture, a ratio of R to S is 1:99 to 99:1.

14. Use of a uracil compound containing a carboxylate fragment of formula (V) as claimed in claim 11 in the preparation of 2-chloro-4-fluoro-5-(3-methyl-2,6-dioxo-4-(trifluoromethyl)-3,6-dihydropyrimidin-1(2H)-yl) benzoate compound of formula (I).

15. Use of an aniline compound containing a carboxylate fragment of formula (IV) as claimed in claim 12 in the preparation of a uracil compound containing a carboxylate fragment of formula (V).

16. Use of a nitrobenzene compound containing a carboxylate fragment of formula (III) as claimed in claim 13 in the preparation of an aniline compound containing a carboxylate fragment of formula (IV).
